# EUROPEAN PATENT SPECIFICATION

(11) **EP 2 679 572 B1**
(45) Date of publication and mention of the grant of the patent: **13.12.2017**
(21) Application number: 12382265.2
(22) Date of filing: 29.06.2012
(51) Int. Cl.: C07C 68/06, C07C 68/08, C07C 69/96, B01D 3/14, B01D 3/00

(54) **Method and apparatus for the production of diaryl carbonate**
Verfahren und Vorrichtung zur Herstellung von Diarylcarbonat
Procédé et appareil pour la production de carbonate de diaryle

(43) Date of publication of application: 01.01.2014
(73) Proprietor: SABIC Global Technologies B.V., 4612 PX Bergen op Zoom (NL)
(72) Inventor: Vic Fernandez, Ignacio, 30151 Murcia (ES); Ferrer Nadal, Sergio, 30390 Murcia (ES); Caballero, Jose Antonio, 03540 Alicante (ES); Javaloyes Antón, Juan, 03201 Alicante (ES)
(74) Representative: Carpintero Lopez, Francisco

(56) References cited:
- US-A1- 2004 266 974
- US-A1- 2009 137 832
- US-A1- 2010 010 252
- US-A1- 2010 261 928
- US-A1- 2012 101 247
- ÖMER YILDIRIM ET AL: "Dividing wall columns in chemical process industry: A review on current activities", SEPARATION AND PURIFICATION TECHNOLOGY, vol. 80, no. 3, 1 August 2011 (2011-08-01) , pages 403-417, XP55043075, ISSN: 1383-5866, DOI: 10.1016/j.seppur.2011.05.009

## Description

### BACKGROUND

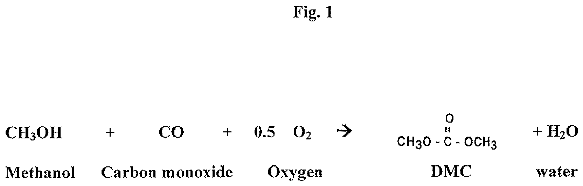

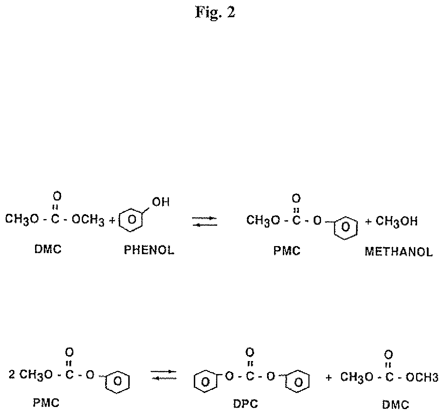

The present disclosure relates generally to a method and an apparatus for the production of diaryl carbonate optionally integrated with a plant for the production of dialkyl carbonate via oxidative carbonylation of an alkyl alcohol, and especially to a method and an apparatus for the production of diphenyl carbonate (also referred to as "DPC") integrated with the production of dimethyl carbonate ("DMC") as starting raw material for the production of said diphenyl carbonate.
Diaryl carbonate, such as DPC, is an important reactant in the production of polycarbonates. For example, polycarbonates can be manufactured by reacting Bisphenol A with DPC. Polycarbonates are useful materials valued for their physical and optical properties. As the uses to which polycarbonates are put have increased, the efficient production of diaryl carbonates has become of greater significance. Early processes for the production of diaryl carbonates utilized phosgene as a reactant. The toxicity of phosgene, however, prompted the development of a non-phosgene process based on the production of an intermediate dialkyl carbonate. The non-phosgene process to produce a diaryl carbonate requires the production of a dialkyl carbonate, such as dimethyl carbonate, by oxidative carbonylation of an alkyl alcohol, such as methanol in the case of DMC production (FIG. 1). Next, the production of diaryl carbonate involves the two-step process depicted in FIG. 2. First, the dialkyl carbonate, such as dimethyl carbonate reacts with an aromatic alcohol, such as phenol, to produce an alkyl aryl carbonate (e.g., phenyl methyl carbonate) and an alkyl alcohol (alkanol), such as methanol, in the presence of a transesterification catalyst. Then, in the second step, two molecules of the alkyl aryl carbonate undergo a disproportionation reaction to produce one molecule of diaryl carbonate, such as DPC and one molecule of dialkyl carbonate, such as DMC. The reaction shown in FIG. 2 is the known desired reaction, but as also is known there are a number of side reactions that occur, producing unwanted by-products. These by-products can interfere with continuing production of the desired product, reduce the efficiency of the overall process, and in some cases produce waste streams requiring special handling for disposal. This is the case of alkyl aryl ethers, such as anisole, which is a side product produced by the reaction of dialkyl carbonate and aromatic alcohol. Other side-reactions, such as the rearrangement of aryl alkyl carbonates or aryl aryl carbonates, might produce other by-products of high boiling points. Thus, a challenge is the development of a method that can minimize the quantities and effects of the reaction by-products, while providing a good yield of the desired product, specifically DPC. U.S. Patent 6,294,684 discloses a method and an apparatus for the production of diaryl carbonate, such as DPC, using a series of distillation columns. Despite these advances, however, there is a continued need for methods and apparatuses exhibiting reduced energy consumption and requiring lower investment costs by having less processing equipment, specifically fewer distillation columns, while also capable of producing purified diaryl carbonate, especially purified DPC, with minimized reaction by-products.

U.S. Patent Application Number 2004/0266974 discloses a method and apparatus for continuously producing an alkyl aryl ether and a diaryl carbonate by reacting a dialkyl carbonate and an aromatic alcohol in presence of a transesterification catalyst.

U.S. Patent Application 2010/0010252 discloses a process for preparing diaryl carbonates from dialkyl carbonates and aromatic hydroxyl compounds using at least two reaction columns, a process section for recovering the dialkyl carbonate used in the reaction and for removing the alcohol of reaction, one or more process steps for removing the by-products obtained in the process which have a boiling point between that of the dialkyl carbonate and that of the alkyl aryl carbonate formed during the preparation of the diaryl carbonate, and a process step for further purification of the diaryl carbonate obtained from the reaction columns.

U.S. Patent Application 2009/0137832 discloses a process comprising transesterifying a dialkyl carbonate and an aromatic hydroxyl compound in the presence of a transesterification catalyst to provide a diaryl carbonate product comprising the transesterification catalyst as an impurity; subjecting the diaryl carbonate product to distillation in a first distillation column having an upper part and a lower part, wherein the upper part comprises a rectifying section and the lower part comprises a stripping section; and withdrawing a first sidestream from the first distillation column, wherein the first sidestream comprises a purified diaryl carbonate.

U.S. Patent Application 2012/0101247 a process for continuously preparing diaryl carbonates from dialkyl carbonates and at least one monohydroxyl compound in the presence of catalysts, and to the use thereof for preparation of polycarbonates.

U.S. Patent Application 2010/0261928 discloses a process for preparing diaryl carbonates and/or alkyl aryl carbonates from dialkyl carbonates and aromatic hydroxy compounds using a reactive dividing wall column.

### BRIEF DESCRIPTION

Disclosed in various embodiments are methods and apparatuses for the production of diaryl carbonate, especially DPC.

In an inventive embodiment, a method for production of diaryl carbonate by reaction of a dialkyl carbonate and an aromatic alcohol in the presence of a transesterification catalyst, comprising: producing a first top stream (5) comprising dialkyl carbonate and alkyl alcohol from a first reactive distillation column (210);introducing a reactant stream (5') comprising the first top stream (5) to a first divided wall distillation column (DWC 1 200) and separating the mixture with use of the first divided wall distillation column (DWC1 200); recovering from the first reactive distillation column (210) a first bottom stream (6) comprising alkyl aryl carbonate; and introducing the first bottom stream (6) into a second reactive distillation column (320) to produce a second bottom stream (15) comprising diaryl carbonate;recovering from the second reactive distillation column (320) the second bottom stream (15) comprising diaryl carbonate and alkyl aryl carbonate, and a second top stream (16) comprising aromatic alcohol and dialkyl carbonate, and recycling the second top stream (16) to the first reactive distillation column (210); recovering from the first reactive distillation column (210) a first side stream (13) comprising dialkyl carbonate, and introducing the first side stream (13) to a first rectification column (810); recovering from the first rectification column (810) a third bottom stream (12) comprising alkyl aryl ether and a third top stream (14) comprising dialkyl carbonate and alkyl alcohol, and recycling the third top stream (14) to the first reactive distillation column (210);recovering from the first divided wall distillation column (DWC1 200) an azeotrope fourth top stream (431) and recycling the azeotrope fourth top stream (431) to the crude product separation column (160); and recovering a dialkyl carbonate recycle stream/fourth bottom stream (426) comprising a purified dialkyl carbonate from the first divided wall distillation column (DWC 1 200) and recycling the dialkyl carbonate recycle stream (426) to the first reactive distillation column (210); recovering a second stream (416) comprising dialkyl carbonate, water and alkanol from a side of the first divided wall distillation column (DWC1 200) and introducing the second stream (416) into a decanter (610); and recovering from the decanter (610) a third stream (418) comprising dialkyl carbonate and recycling the third stream (418) to the first divided wall distillation column (DWC1 200).

In an inventive embodiment, an apparatus for continuous production of diaryl carbonate by reaction of a dialkyl carbonate and an aromatic alcohol in the presence of a transesterification catalyst, the apparatus comprising: a first reactive distillation column (210), a second reactive distillation column (320), a first rectification column (810), a first divided wall distillation column (DWC1 200) and a plurality of lines for transporting reactant and product streams; wherein the first reactive distillation column (210) is connected to input lines for introduction of reactants, and to a first transfer line, a second transfer line, and a third transfer line, the first transfer line extends from the top of the first reactive distillation column (210) to an inlet of the first divided wall distillation column (DWC 1 200), the second transfer line extends from the bottom of first reactive distillation column (210) to an inlet of the second reactive distillation column (320) without passing through another reactive distillation column, and the third transfer line extends from the side of the first reactive distillation column (210) to an inlet of the first rectification column (810); wherein the second reactive distillation column (320) is connected to a first recycle line extends from the top of the second reactive distillation column (320) to an inlet of the first reactive distillation column (210), and a first product line extends from the bottom of the second reactive distillation column (320) for providing diaryl carbonate; wherein the first rectification column (810) is connected to a fourth transfer line, the fourth transfer line extends from the top of the first rectification column (810) to an inlet of the first reactive distillation column (210); and wherein the first divided wall distillation column (DWC 1 200) is connected to a second product line for providing dialkyl carbonate/alkyl alcohol azeotrope extends from the top of the first divided wall distillation column (DWC 1 200) through a crude product separation column (160) to an inlet of the first reactive distillation column (210), a middle height side draw line extends from a middle of the first divided wall distillation column (DWC1 200) to a decanter (610), a third line that extends from the decanter (610) to the first divided wall distillation column (DWC 1 200), and a second recycle line extends from the bottom of the first divided wall distillation column (DWC1 200) to an inlet of the first reactive distillation column (210). The first rectification column (810) can be connected to a fourth transfer line, the fourth transfer line extends from the top of the first rectification column (810) to an inlet of the first reactive distillation column (210). The first divided wall distillation column (DWC1 200) can be connected to a second product line for providing dialkyl carbonate/alkyl alcohol azeotrope extends from the top of the first divided wall distillation column (DWC1 200) through a crude product separation column (160) to an inlet of the first reactive distillation column (210), and a second recycle line extends from the bottom of the first divided wall distillation column (DWC1 200) to an inlet of the first reactive distillation column (210).

The above described and other features are exemplified by the following figures and detailed description.

### BRIEF DESCRIPTION OF THE DRAWINGS

Refer now to the figures, which are exemplary and non-limiting embodiments, and wherein the like elements are numbered alike.
FIG. 1 schematically depicts the synthesis reaction of dimethyl carbonate via oxidative carbonylation of methanol in the presence of a suitable catalyst.
FIG. 2 schematically depicts the two-step reaction of dimethyl carbonate and phenol (PhOH) to produce diphenyl carbonate.
FIG. 3 schematically depicts a plant design for the production of diaryl carbonate, such as DPC.
FIG. 4 schematically depicts a reaction section of a dialkyl carbonate plant design for producing an effluent feed stream into column 160 shown in FIG. 3.
FIG. 5 schematically depicts a plant design for the separation of DMC, water and methanol.
FIG. 6 depicts a mass balance diagram of a DMC/methanol/water ternary system.
FIG. 7, being outside the claimed scope, schematically depicts a plant design for the production of diaryl carbonate, such as DPC.

### DETAILED DESCRIPTION

The disclosure herein is directed to how to improve the overall efficiency, reduce energy consumption and reduce the overall cost associated with the production of dialkyl carbonate and diaryl carbonate, especially DMC and DPC through efficient integration of the process plants to manufacture these two monomers. The number of sequential distillation columns needed to produce and purify DPC starting from DMC can be reduced, e.g., without changing the other equipment or changing flow rates. With the reduction in equipment, the processing equipment can be redesigned and processing parameters regarding, for example, pressure and temperature can be adjusted.

DMC and DPC production facilities are integrated by, for example, merging a distillation column from DPC production and a distillation column from DMC production into a single divided wall distillation column. The use of this divided wall distillation column not only merges two columns thereby reducing processing equipment, but also can alleviate the need for a DMC recovery column in the DMC production portion of the facility thereby further reducing energy consumption of the entire system. If a second divided wall distillation column is employed in the DPC recovery section of the facility, an even further reduction in processing equipment and further energy savings can be achieved. A proper concentration range for the reactants entering each divided wall distillation column can be identified and achieved in order to result in a proper separation of the constituents. As a result of the present system, a surprising reduction in energy consumption and cost during DPC production.

A more complete understanding of the components, processes, and apparatuses disclosed herein can be obtained by reference to the accompanying drawings. These figures (also referred to herein as "FIG.") are merely schematic representations based on convenience and the ease of demonstrating the present disclosure, and are, therefore, not intended to indicate relative size and dimensions of the devices or components thereof and/or to define or limit the scope of the embodiments. Although specific terms are used in the following description for the sake of clarity, these terms are intended to refer only to the particular structure of the embodiments selected for illustration in the drawings, and are not intended to define or limit the scope of the disclosure. In the drawings and the following description below, it is to be understood that like numeric designations refer to components of like function.

A principle reaction adjusted herein is the afore-described reaction set forth in FIG. 2. It is noted that this reaction can be carried out using various dialkyl carbonates and various aromatic alcohols, such as those disclosed, for example, in U.S. Patent Nos. 5,210,268, 5,344,954, 5,705,673, and 6,294,684. Examples of dialkyl carbonates are those that include a carbonate group disposed between two alkyl groups. Specific examples include dimethyl carbonate, diethyl carbonate, di-n-propyl carbonate, di-isopropyl carbonate, di-n-butyl carbonate, and dihexyl carbonate. An example of an aromatic alcohol is phenol, and further specific examples of aromatics include unsubstituted phenol o-, m- or p-cresol, o-, m- or p-chlorophenol, o-, m- or p-methoxyphenol, 2,6-dimethylphenol, 2,4-dimethylphenol, 3,4-dimethylphenol, 1-napthol, and 2-naphthol.

Particularly desirable reactants useful on an industrial scale are DMC and phenol, which react to produce DPC. It is noted that these specific reactants will be primarily referred to below in the description for ease of reference and merely as non-limiting and exemplary materials. It should be understood that this usage is merely for ease of reference and that no limitation on embodiments disclosed herein to only the specific use of these materials is intended. The other materials set forth above are also contemplated.

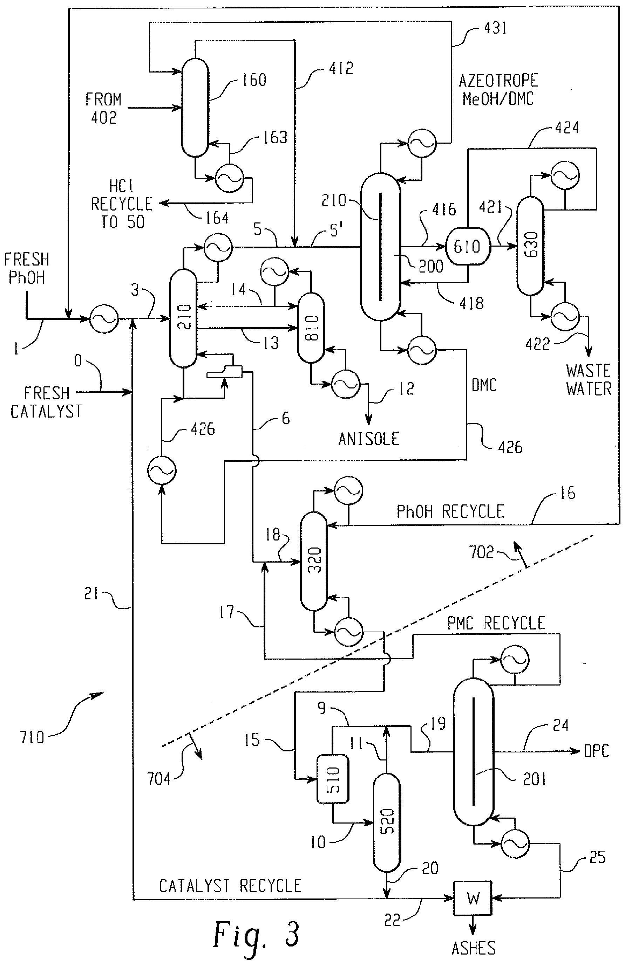

FIG. 3 schematically depicts a diaryl carbonate production plant/recovery apparatus/design 710, comprising a reaction/distillation section 702 and a purification section 704.

Various feed, product, and recycle streams also are shown in FIG. 3. It is further noted that it will be appreciated by persons skilled in the art that the positioning of the various streams/lines as described herein as being, e.g., in the "top", "middle", "bottom" or "side" of a particular column is relative because the actual position at which material is to be introduced or recovered is dependent on the conditions being maintained in the particular column, For example, a stream entering the "bottom" of a column may actually enter several stages above the sump including the reboiler of the column, and a line/stream exiting the "top" of the column may actually exit several stages below the top stage including the condenser of the column. Thus, such terms herein are included for ease of reference to describe a general orientation regarding various columns and lines/streams and such terms are not meant to be limiting to one exact location. Also, the columns referred to herein can be interconnected by a series of feed/recycle lines which serve to transport streams comprising reactants and/or products. The direction of flow for each line is indicated on FIGS. 3-5. Various valves, heaters, pumping equipment, instruments, filters, analyzers and other fittings, optionally, can be included with the lines shown therein in adapting the design to a particular installation. Also, although for illustrative purposes, the Figures and their description may depict singular vessels, such as reaction vessels or mixing vessels, it is understood that multiple vessels in series or parallel may be used where suitable.

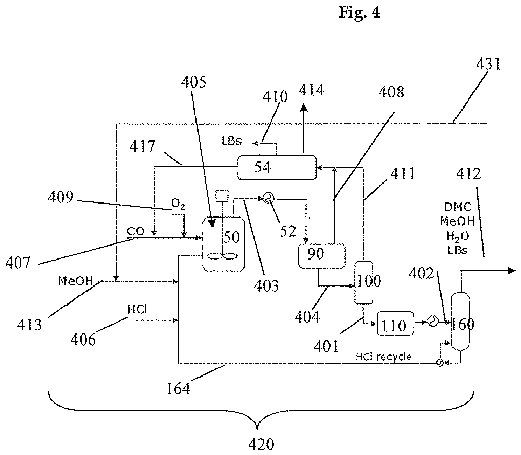

As shown in FIG. 3, a reactant effluent stream 402 can enter crude product separation column 160, which can optionally also serve as an acid removal column. Stream 402 can be produced in the reaction section 420 shown in FIG. 4 of an exemplary DMC production facility. As described in detail below, it is now possible to integrate portions of an exemplary DMC production facility and DPC production facility using, e.g., divided wall distillation column(s) to reduce processing equipment and energy consumption.

Stream 402 of FIG. 3 can be produced utilizing the reaction section 420 as depicted in FIG. 4 as follows. With reference to the reaction section 420 of FIG. 4, O₂ stream 409 comprises oxygen (O₂), which can be provided in any form, such as gaseous form. Oxygen sources include, for example, air, or oxygen-containing gases, including those having greater than or equal to 95 wt% molecular oxygen, specifically greater than or equal to 99 wt% molecular oxygen. Oxygen-containing gases are commercially available from, for example, Air Products.

CO stream 407 comprises carbon monoxide (CO) which can be provided in any form, such as gaseous form. Carbon monoxide sources include, for example, carbon monoxide, carbon dioxide mixtures, syngas and/or other carbon monoxide-containing gases and combinations thereof, including those having greater than or equal to 95 wt% (wt%) molecular carbon monoxide, specifically greater than or equal to 99 wt% molecular carbon monoxide.

Stream 413 (first alkanol stream) comprises alkanol, such as methanol (MeOH). It is noted, however, that other alkanols could be used. Examples of alkanols include primary, secondary and tertiary C₁-C₁₂ alkanols, specifically, primary C₁-C₆ alkanols.

The first alkanol stream 413, oxygen stream 409 and carbon monoxide stream 407 can be added to a reactor (e.g., stirred tank reactor 50), e.g., in a molar ratio of 0.5 to 0.7 (alkanol): 0.04 to 0.06 (oxygen): 0.8 to 1.2 (carbon monoxide), respectively and specifically, greater than or equal to 0.6 (alkanol): greater than or equal to 0.05 (oxygen): greater than or equal to 1 (carbon monoxide).

Catalyst stream 405 comprising a catalyst can also be added to stirred tank reactor upon demand, and/or the catalyst can remain in the stirred tank reactor 50. Examples of catalysts include those capable of catalyzing the reactants entering reactor 50 to produce a dialkyl carbonate formation reaction as is known in the art. The catalyst can comprise iron, copper, nickel, cobalt, zinc, ruthenium, rhodium, palladium, silver, cadmium, rhenium, osmium, iridium, platinum, gold, mercury, and so forth, and combinations comprising at least one of the foregoing metals. As an example, the catalyst can comprise copper and chloride ions in a molar ratio of Cu:Cl of greater than or equal to 0.1, specifically, 0.1 to 3.0, more specifically, 0.5 to 3.0, yet more specifically, 0.8 to 3.0, and even more specifically, 1.2 to 3.0. Tthe molar ratio of Cu:Cl of can be 0.5 to 1.5. Specific catalysts include cuprous chloride (CuCl) and cupric chloride (CuCl₂). It is noted that the amount of catalyst used relative to the reactants (e.g., alkanol, carbon monoxide, oxygen, and hydrochloric acid) can depend on the particular catalyst employed. The catalyst concentration should be sufficiently high to produce an acceptable yield, but should be kept below a concentration that would cause solid setting of the catalyst in the reactor 50 or the clogging of equipment. For example, when the catalyst comprises CuCl, the catalyst concentration can be 50 to 250 grams/liter of total liquid reaction volume of the mixture in the reactor 50, especially 80 to 180 grams/liter of total liquid reaction volume of the mixture in reactor 50.

During operation of the DMC production process, an effective chloride ion concentration can be maintained by the addition hydrochloric acid (HCl) from HCl stream 406.

Sufficient HCl from stream 406 can be added from a HCl tank (not shown) during the course of the reaction to maintain a molar ratio of Cu:Cl of greater than or equal to one, for example. The concentration of HCl can be continuously determined and controlled by the addition of, e.g., fresh HCl (stream 406). A typical mass ratio for HCl feed to total liquid feed (e.g., the sum of streams 431+413+406+164) entering reactor 50 can be 4 x 10⁻⁴ to 4 x 10⁻³.

Streams 406, 413, 407, 431, 417, 164, and 409 can enter the reactor 50, as shown in FIG. 4, where the constituents react in the presence of catalyst 405 to produce a stream 403 (second alkanol stream) exiting the reactor 50. The catalyzed reaction of alkanol, oxygen, and carbon monoxide may be performed in a single reactor 50, or in multiple reactor(s) 50. The conditions in reactor(s) 50 can be selected to balance the maximizing of the yield of dialkyl carbonate while minimizing the degradation of dialkyl carbonate. The reaction can be performed, for example, at a temperature of 50°C to 250°C. Within this range, the temperature can be greater than or equal to 100°C. Also within this range, the temperature may be less than or equal to 150°C. The reactor 50 can be maintained at a pressure of 15 bar gauge (barg) to 35 barg (e.g., 1,500 kilopascal (kPa) to 3,500 kPa). Within this range, a pressure of greater than or equal to 20 barg (2,000 kPa) can be employed. Also within this range, a pressure less than or equal to 28 barg (2,800 kPa) can be employed. In the case of dual reactor systems, the catalyst may be recycled between tanks.

Stream 403 can comprise dialkyl carbonate, alkyl chloroformate, hydrochloric acid, water, carbon monoxide, alkanol, oxygen, inert gases coming with fresh oxygen and carbon monoxide streams, as well as side products, such as alkyl chlorides and dialkyl ethers. Examples of dialkyl carbonates are those that include a carbonate group disposed between two alkyl groups. The dialkyl carbonate formation reaction will create a dialkyl carbonate that is dependent upon the alkanol used as a reactant. Thus, if methanol is used as a reactant in stream 413, for example, the dialkyl carbonate will comprise dimethyl carbonate, and so forth.

Stream 403 is typically withdrawn from the reactor 50 in a gas/vapor form. The term "vapor" herein refers to gaseous organic components of the mixture, such as for example, evaporated dialkyl carbonates, alcohols, alkyl chloroformates, and so forth, and to water vapor. That is, the term "vapor" refers to fluids having a boiling point of greater than or equal to -50°C at atmospheric pressure. In contrast, the term "gas" herein refers to, e.g., gaseous oxygen, carbon dioxide, carbon monoxide and optional nitrogen. That is, the term "gas" refers to such constituents having a boiling point of less than -50°C at atmospheric pressure. The vapor can be at least partially condensed in a condenser 52 (e.g., greater than or equal to 10 wt% condensed) or more fully condensed (e.g., greater than or equal to 90 wt% condensed) and fed to a first gas-liquid separator 90. The reaction section 420 can optionally employ a single gas-liquid separator or a plurality of (i.e., greater than or equal to 2; specifically, 2 to 5) gas-liquid separator(s). The first gas-liquid separator 90 can be maintained at a pressure within 10 percent (%), specifically within 1%, of the pressure of the reactor 50. The gas effluent from the first gas-liquid separator 90 (e.g., flash separator) can be recycled in stream 408, which comprises carbon monoxide, to reuse excess carbon monoxide and return it as stream 417 to the initial carbon monoxide feed of stream 407. Stream 408 can also comprise unreacted carbon monoxide and oxygen, by-product carbon dioxide, and low boiling point by-products (low boiling point organics). Stream 408 can enter a separation section 54, from which stream 417 can be merged with stream 407 to return excess carbon monoxide to reactor 50. Optionally, all or part of stream 408 can bypass separation section 54 and merge directly into Stream 417. Stream 410 is a purge stream from the separation section 54, comprising low boiling by-products (low boiling point organics). Specifically, the low boiling point organics, as referred to herein, can comprise organic compounds having a boiling point less than or equal to 65°C, such as, for example, methylal, dimethyl ether, methyl chloride, and a combination of any of the foregoing. Stream 414, comprising carbon monoxide and carbon dioxide free from low boiling point organic compounds (low boiling point organics), can be sent to a proper treatment unit to recover and reutilize the carbon monoxide.

Stream 404 comprising dialkyl carbonate, alkyl chloroformate, hydrochloric acid, water, low boiling point organics, and unreacted alkanol and carbon monoxide, can then exit the separator 90, as shown in FIG. 4, and enter a second gas-liquid separator 100 (e.g., flash separator). The second gas-liquid separator 100 can be maintained at a pressure less than or equal to 20% of the pressure of reactor 50 (e.g., less than or equal to 3 bar gauge (300 kPa), specifically less than or equal to 0.2 bar gauge (20 kPa)) to achieve a separation of greater than or equal to 90 wt%, specifically greater than or equal to 95 wt%, of gas in stream 404. Optionally substantially all of the gas is removed from the mixture (e.g., greater than or equal to 99 wt%). The gas effluent removed from the second gas-liquid separator 100 can also be recycled. The vapor in the mixture can be in an at least partially condensed form (i.e., greater than or equal to 10 wt% condensed), especially a fully condensed form (i.e., greater than or equal to 90 wt% condensed), before entering the first gas-liquid separator 90, and/or between the first gas-liquid separator 90 and the second gas-liquid separator 100. The vapor in stream 404 can be at least partially condensed (e.g., greater than or equal to 10 wt%) or more fully condensed (e.g., greater than or equal to 90 wt%) prior to entering the second gas-liquid separator 100). Optionally, all of the gas can be removed in the second gas-liquid separator 100. The gas effluent from the second gas-liquid separator 100, stream 411, comprising unreacted carbon monoxide, carbon dioxide and other low boiling point components (low boiling point organics), also can be optionally treated in separation unit 54 to recover unreacted carbon monoxide. Stream 401 exiting the second gas-liquid separator 100 can comprise a single liquid phase comprising dialkyl carbonate, residual methanol, water, hydrochloric acid, alkyl chloroformate, and low boiling point organics. The alkyl chloroformate is then removed from the stream 401. For example, stream 401 can enter fluid passageway 110 configured to remove alkyl chloroformate by decomposition into methanol, carbon dioxide, dialkyl carbonate, and hydrochloric acid. U.S. Patent No. 6,784,277 describes benefits and methods of removing alkyl chloroformate using a fluid passageway.

After removal of the alkyl chloroformate from the stream 401, the stream optionally proceeds, (e.g., as stream 402 comprising dialkyl carbonate, water, low boiling point organics, and hydrochloric acid) through heat exchanger 150 to at least partially vaporize stream 402. Heat exchanger 150 can have a residence time of less than or equal to 10 minutes. Stream 402 can then enter crude product separation column 160, which optionally serves as an acid removal column, for example to remove HCl. Column 160 also can help remove any entrained catalyst (e.g., CuCl) in stream 403, which could otherwise contribute to downstream corrosion. In the column 160, the vaporized condensate can encounter a counter-flowing liquid supplied by counter-flowing liquid line (not shown), which can comprise an alkanol and dialkyl carbonate azeotrope mixture, to a higher point in the column 160 (e.g., upper third). The counter-flowing liquid can trap remaining HCl, which may be removed, e.g., from the bottom, of column 160 in stream 164 comprising hydrochloric acid, water and small amounts of alkanol and dialkyl carbonate (e.g., less than or equal to 10 weight percent (wt%)). For example, stream 164 can comprise greater than or equal to 6 wt% HCl and/or less than or equal to 15 wt% organic compounds. Hydrochloric acid can be recycled back to reactor 50 with use of HCl recycle stream 164 and merge with fresh HCl feed stream 406. Optionally, a portion of HCl recycle stream 164 can be passed through a heat exchanger and recycled to column 160 as stream 163.

Stream 412 comprising dialkyl carbonate, alkanol (e.g., methanol), water and low boiling point organics (low boiling by-products comprising organics having a boiling point less than or equal to 65°C) can be removed from the top of column 160 and advantageously passed into a first dividing wall distillation column (DWC1) 200, as shown in FIG. 3 by mixing with stream 5 from, e.g., the distillate of column 210 to form stream 5' which enters DWC1 200.

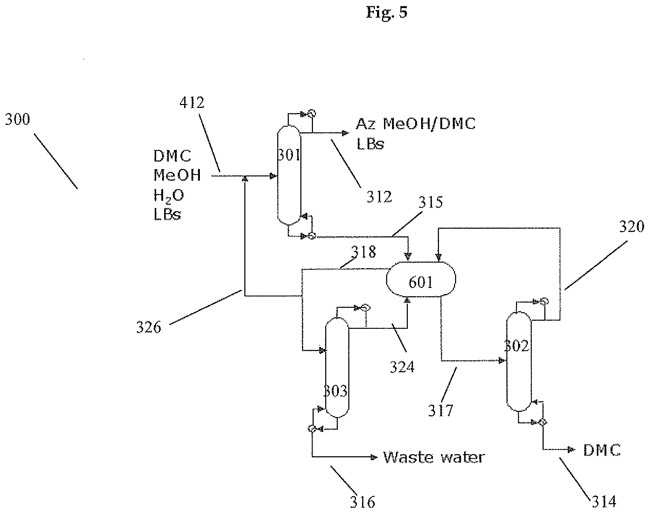

The use of a dividing wall distillation column such as DWC1 200 can reduce the number of distillation columns needed to produce dialkyl carbonate, such as DMC, while maintaining the purity level (e.g., greater than or equal to 99.8 wt% purity), thereby significantly reducing the plant investment and overall cost of production, and without increasing energy consumption. For example, FIG. 5 schematically depicts a DMC/methanol/water separation system 300 comprising three distillation columns: a first distillation column 301, a second distillation column 302, and a third distillation column 303. FIG. 5 also depicts a separator 601. This separator 601 is intended to be a unit to separate an aqueous-rich phase and an organic-rich phase and it can be for example a decanter, pervaporation unit, and so forth. Separator 601 is referred to herein as decanter 601 in a non-limiting example of this separator. The use of DWC1 200 can eliminate the need for at least the DMC recovery distillation column 302 of FIG. 5, as described in further detail below, thereby reducing the energy consumption of the entire system and reducing equipment and production costs.

With reference to FIG. 5, a mixed feed stream 412 comprising dialkyl carbonate, alkanol (alkyl alcohol), water and low boiling point organics can be removed from the top of column 160 (shown in FIG. 4). As shown in FIG. 5, stream 412 can thus comprise methanol (MeOH), DMC, water, and low boiling point organics, and can be separated into three product streams comprising: mixture of alkanol/dialkyl carbonate azeotrope (methanol/DMC azeotropic composition) and low boiling point organics (stream 312), dialkyl carbonate (e.g., DMC, stream 314), and water (stream 316). Feed stream 412 can enter a first distillation column 301, which can distill out a product stream 312 comprising an azeotropic composition of alkanol and dialkyl carbonate (i.e., azeotrope methanol/dimethyl carbonate, and the low boiling point organics). The stream 315 exiting column 301, which can comprise a mixture of dialkyl carbonate (e.g., DMC) and water, with small amounts of alkanol (methanol), can enter decanter 601. Stream 315 can be decanted into a dialkyl carbonate (e.g., DMC) rich stream 317 (organic phase) and a water rich (light aqueous phase) stream 318. Stream 317 comprising dialkyl carbonate (e.g., DMC) can enter dialkyl carbonate recovery column 302 (second distillation column) where stream 320 comprising dialkyl carbonate (e.g., DMC), some alkanol (e.g., methanol), low boiling point organics, and water, can be condensed and recycled to the decanter 601. Also exiting column 302 is product stream 314 comprising DMC.

Stream 318 exiting the decanter 601 can be fed to waste water recovery column 303 (third distillation column). From column 303, azeotrope DMC/water can be recycled, e.g., from near the top (distillate of the column), in stream 324 to the decanter 601, and waste water stream 316 can be removed, e.g., the bottom residue stream of column 303. Bleedstream 326 comprising water, dialkyl carbonate, alkanol, and low boiling point organics coming off of stream 318 can be fed back to column 301, as shown in FIG. 3. Use of stream 326 can help avoid buildup of low boiling point organics and methanol in decanter 601.

Thus, while the above-described process and design of FIG. 5 can be employed to produce DMC, three distillation columns 301, 302 and 303 are needed. Moreover, it is noted that gradually purer methanol can be obtained from column 301 overheads (stream 312) by progressively increasing the operating pressure of column 301. In this method, a stream 312 comprising 93 wt% methanol, for example, can be obtained from column 301 in stream 312, with column 301 operating at a pressure greater than or equal to 8 bar gauge (800 kPa), for example. Thus, stream 312 comprising a greater weight percent methanol (purer methanol) can be obtained by operating at a higher pressure, but at the cost of also using higher pressure steam as a heating medium in the process. Moreover, an additional drawback if column 301 operates at higher pressure is that an increase in the number of theoretical stages (e.g., trays) in the column 301 would be needed to minimize the presence of methanol in the exiting stream 315 sent to decanter 610. It would thus be desirable to reduce at least one of the columns 301, 302, 303 and increase the overall energy efficiency of the production, as well as reduced the costs associated with the DMC production.

Tthe need for at least column 302 can be eliminated while producing a purified dialkyl carbonate product (e.g., dialkyl carbonate having a purity of greater than 99.9 wt%), specifically purified DMC (impurities in trace amounts), in integrated DPC and DMC production facilities.

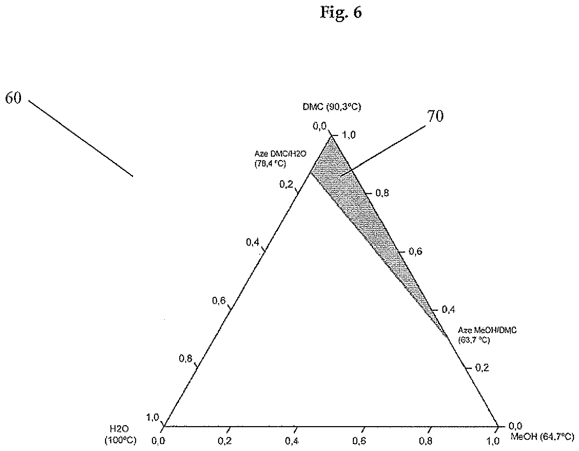

As shown in FIG. 3, effluent reactant stream 412 can enter DWC1 200. A separation is carried out of, e.g., a mixture comprising DMC, methanol and water, which also can include some low boiling point organics, into pure DMC (impurities in trace amounts, e.g., less than 0.01 wt% impurity based upon a total weight of the DMC), an azeotropic mixture of MeOH/DMC with low boiling point organics and a water stream. This separation can be effectively accomplished for mixture feeds (e.g., stream 412) having a composition, for example, within the triangle 70 formed in a ternary composition diagram between three vertices: pure DMC, azeotrope methanol/DMC and azeotrope DMC/water, shown in FIG. 6. FIG. 6 depicts a mass ternary composition diagram 60 of a DMC/methanol/water ternary system. The shadowed triangle 70 shows examples of effective ternary feeds at atmospheric pressure. More specifically, the compositional ranges of the feed can comprise, wt% for example, 100 wt% DMC (pure dimethyl carbonate); 68 wt% methanol, and 32 wt% DMC (methanol/DMC azeotrope); and 87 wt% DMC and 13 wt% water (azeotrope DMC/water). In other words, the compositional ranges lie within region 70, the area of the ternary diagram identified by coordinates: 100 wt% DMC; 68 wt% methanol and 32 wt% DMC; and 87 wt% DMC and 13 wt% water. The amount of the referenced low boiling point organics is considered low enough not to influence the ternary equilibrium of the DMC/methanol/water system.

Thus, as shown in FIG. 3, effluent reactant stream 412 can exit column 160, mix with stream 5 from column 210 to form stream 5' and enter DWC1 200. Stream 5' can comprise dialkyl carbonate, water, alkanol and low boiling point organics (low boiling point organics). Specifically, the low boiling point organics can comprise organic compounds having a boiling point less than or equal to 65°C, such as, for example, methylal, dimethyl ether, methyl chloride, and a combination of any of the foregoing. The low boiling point organics are typically present in stream 412 in a total amount less than or equal to 2 wt% based upon the total weight percent of stream 412, specifically less than 1 wt%.

Regarding the structure of DWC1 200, as an example, this dividing wall distillation column can comprise the following sections: a) an inflow section and b) an offtake section, c) an upper column section, and d) a lower column section of, e.g., greater than or equal to four, e.g., greater than or equal to eight, trays, e) a condenser and f) a reboiler. The inflow section and the offtake section can both comprise, greater than or equal to 10 trays, specifically, greater than or equal to 20 trays, more specifically, greater than or equal to 26 trays, and can be separated by a wall (e.g., a vertical wall). The upper column section can have greater than or equal to four, specifically, greater than or equal to eight trays. The lower column section can have greater than or equal to 4 trays, specifically, greater than or equal to 8 trays. As explained by Schultz et al., Chem. Eng. Process., May 2002, pages 64-70, a basis for a dividing wall column concept is the Petlyuk's configuration but with the two distillation columns installed within a single shell.

A divided wall distillation column, such as DWC1 200 in FIG. 3, can comprise a dividing wall 210, as schematically shown in FIG. 2, which can vertically bisect a portion of the interior of the column 200. It is not necessary for the dividing wall 210 of the dividing wall distillation column 200 to extend all the way to the top and/or bottom sections of the column 200, as shown in FIG. 3, thus enabling the dividing wall distillation column 200 to be reboiled and refluxed. Such a divided wall distillation column 200 can enable an inlet stream to enter on, e.g., one side of the dividing wall 210 of the column 200, and one or more exiting streams, e.g. side drawn streams, can be located on the other side of the dividing wall 210. Such a design can increase the stability of the column 200 and enable one or more product streams of differing composition to exit the dividing wall column 200.

With further reference to FIG. 3, a stream 431 comprising alkanol, dialkyl carbonate, water and low boiling point organics can be recovered from, e.g., the top (distillate) of DWC1 200. Specifically, the low boiling point organics in this stream can comprise organic compounds having a boiling point less than or equal to 65°C, such as, for example, methylal, dimethyl ether, methyl chloride, and a combination of any of the foregoing. The low boiling point organics can be present in stream 431 in a total amount less than or equal to 2 wt% based upon the total weight percent of stream 414, specifically less than 1 wt%. Stream 431 also can comprise DMC/methanol azeotrope. Stream 431 comprising alkanol and dialkyl carbonate can be partly recycled back to the original alkanol feed stream 413 to the reaction section of the integrated dimethyl carbonate production plant, as shown in FIG. 4. A stream 426 comprising dialkyl carbonate, such as pure DMC (impurities only in trace amounts) can be recovered from, e.g., the bottom of DWC1 200. The DWC1 200 can operate at, for example, atmospheric pressure. The temperature profile of column DWC1 200 can be greater than or equal to 60°C, specifically greater than or equal to 70°C. Examples of temperature ranges for the temperature profile in the column DWC1 200 include 60°C to 180°C, specifically 70°C to 140°C. The operating pressure in column DWC1 200 can be greater than or equal to 1 bar (100 kPa), specifically greater than or equal to 1.1 bar (110 kPa). Examples of pressure ranges include 1.2 to 3 millibar (120 to 300 Pa).

Stream 416, which can comprise dialkyl carbonate, water and traces of alkanol, and exiting DWC1 200 from the side, for example, also can be recovered in the process. Stream 416 can enter decanter 610 and be split into two phases: stream 418 (organic phase) and stream 421 (aqueous phase). Stream 418 comprises mostly dialkyl carbonate, some water and traces of alkanol, which is returned back to the DWC1 200, such as to the top stage of the lower section of the column 200. Stream 421 (aqueous phase), which can comprise mostly water, some dialkyl carbonate and traces of alkanol can exit the decanter 610, for example, as a side drawn and be sent to waste water recovery column 630. A purge stream from column 630 can be included such as, for example, a bottom residue stream 422, which can comprise mostly water (e.g., greater than or equal to 95 wt% based upon the total weight of stream 422, specifically, greater than or equal to 99 wt%) and alkanol (e.g.,, less than or equal to 5 wt% based upon the total weight of the stream 422, specifically, less than or equal to 1 wt%). Stream 424 comprising dialkyl carbonate, water, and alkanol from the top of column 630 can optionally be recycled back to the decanter 610. The temperature profile of column 630 can be greater than or equal to 60°C, specifically greater than or equal to 70°C. Examples of temperature ranges for the temperature profile in the column 630 include 60°C to 120°C, specifically 70°C to 110°C. The operating pressure in column 630 can be greater than or equal to 0.5 bar (50 kPa), specifically greater than or equal to 0.8 bar (80 kPa). Examples of pressure ranges include 0.8 to 3 bar (80 to 300 kPa). Product and/or recycle stream 426 comprising pure dialkyl carbonate, such as pure DMC with impurities only in trace amounts, can optionally be recovered from, for example, the bottom of DWC1 200 (recovery not shown in FIG. 3) and/or recycled back as an initial reactant to feed column 210, as described in detail below. Thus, stream 426 also can optionally proceed through a recovery station (not shown) for recovery and collection of, e.g., pure DMC product.

Optionally, a DMC production facility can be integrated with a DPC production facility, which also is explained in more detail below.

More specifically, with further reference to FIG. 3, columns 210 and 320 can be reactive distillation columns. As a non-limiting example, these columns can each have a lower reaction section in which a chemical reaction occurs, and an upper section. In general, the reactive portion of the column can be furnished with packings or fixed internals to provide at least 3 theoretical distillation stages (trays). For example, the reaction section of column 210 can provide greater than or equal to 10, such as 10 to 60, trays, specifically greater than or equal to 15, such as 15 to 40, theoretical distillation stages (trays). Known dumped packings and/or arranged packings can be employed. Specifically, packings having a large surface area, good wetting and residence time of the liquid phase, such as, for example, Novolax rings, CY packings, can be used. Fixed internals, such as tray columns also can be employed, and specific examples include sieve trays, valve trays, bubble-cap trays, and so forth.

Column 810 can be a rectification column. Thus, this column can carry out a separation of materials based upon boiling point, without driving a concurrent chemical reaction. The afore-referenced columns can be interconnected by a series of feed/recycle lines that serve to transport streams comprising reactants and/or products.

With further reference to FIG. 3, Stream 0 can comprise a transesterification catalyst and phenol. Catalysts include titanium compounds, such as, titanium tetraphenoxide, titanium isopropylate, and titanium tetrachloride, organotin compounds such as octylstannoic acid and dibutyltin oxide, and compounds of copper, lead, cobalt, vanadium, zinc, iron, and zirconium. Stream 1 comprises fresh phenol (PhOH). Stream 426, described above, comprises dialkyl carbonate, specifically DMC. Starting materials and/or reactants can be introduced to column 210 through streams 3 and 426. Stream 3 is a combination of streams 0, 1, and 426, and recycle stream 16 and recycle stream 21, discussed in further detail below and stream 0. Stream 21 comprises a catalyst recycle stream. Stream 426 can comprise pure DMC (impurities in trace amounts) and be recovered from, for example, the bottom of the dividing wall distillation column (DWC1) 200 or partially from a storage tank.

Stream 426 can be fed to, for example, the bottom of column 210, specifically to the reboiler. Stream 426 can be a liquid or a vapor, depending upon the type of reboiler used or the point where this stream is fed to the 210 column. Stream 3 can be fed as a liquid into, e.g., the middle section of column 210, more specifically at a location at or near the top of the reactive distillation section. The feed rate of streams 3 and 426 can be such that the molar ratio of dialkyl carbonate to aromatic alcohol, which is introduced into column 210, is between 0.1 and 10, specifically between 0.2 and 5, and more specifically between 0.5 and 3. Dialkyl carbonate can be provided in excess through stream 426 because the dialkyl carbonate can serve as both a reactant and as a stripping agent, which facilitates removal of alkyl alcohol (alkanol) produced in the transesterification reaction. This removal can increase the rate of production of alkyl aryl carbonate in column 210.

A transesterification reaction can be carried out in column 210 at, for example, a temperature greater than or equal to 100°C, specifically greater than or equal to 130°C, and more specifically greater than or equal to 140°C. Examples of temperature ranges for carrying out the reaction include, 100°C to 300°C, specifically 110°C to 275°C, and more specifically 120°C to 250°C. The operating pressure at the top of column 210 can be greater than or equal to 0.5 bar (50 kPa), specifically greater than or equal to 3, and more specifically greater than or equal to 5 bar (500 kPa).

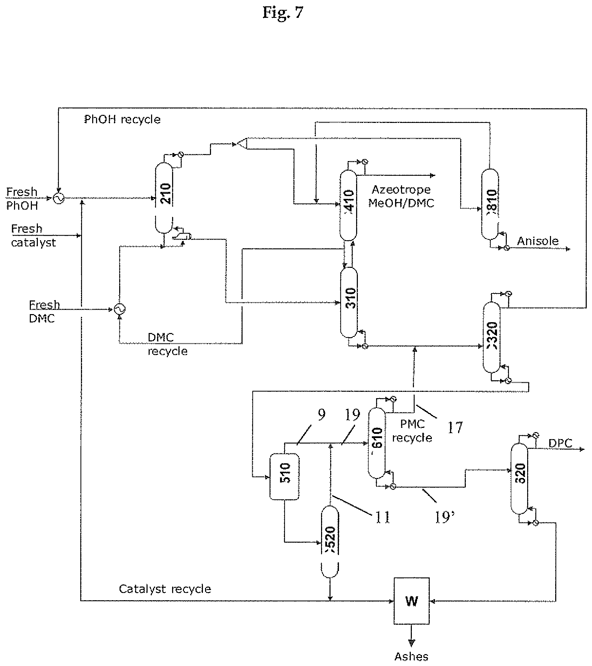

Reactant products and unreacted starting materials can be removed from column 210 through streams 5 and 6, for example in a continuous manner. Stream 5, which can be drawn from, e.g., the top of column 210, can comprise unreacted dialkyl carbonate and also can comprise the alkyl alcohol produced in the transesterification reaction. Stream 5 can then pass to divided wall distillation column (DWC1) 200, described above. As disclosed herein, the need for, e.g., rectification column C410 in a configuration of a DPC plant, as shown in, e.g., FIG. 7 herein, can be eliminated, as well as eliminate the rectification column described in U.S. Patent No. 7,141,641, as further described below.

FIG. 3 contemplates the combination of embodiments such as a column, e.g., C410 shown in FIG. 7 of an exemplary DPC plant, and a column such as C301 shown in FIG. 5 of a DMC unit into just one divided wall column (DWC1 200) in FIG. 3. While a column such as C410 can be effective, exemplary embodiments described herein allow for the integration of DPC and DMC production facilities with use of a divided wall distillation column (DWC1 200), thus avoiding the utilization of processing equipment, e.g., C410.

Stream 6, which can be drawn from, e.g., the bottom of column 210, can comprise the alkyl aryl carbonate produced in column 210, in combination with unreacted starting materials and catalyst. Stream 6 can be augmented by the addition of a recycle stream (stream 17), which can comprise alkyl aryl carbonate to form stream 18. Stream 17 can be an alkyl aryl carbonate and aromatic alcohol containing stream recovered from the purification section 704 during optional purification of dialkyl carbonate in purification section 704, which is described in further detail below. Such augmentation can result in an improvement in the overall production of diaryl carbonate. Thus, stream 6, now augmented with stream 17 to form stream 18 and comprising alkyl aryl carbonate, can according to some embodiments enter the second reactive distillation column 320, without passing through another reactive distillation column, to produce diaryl carbonate by disproportionation of the alkyl aryl carbonate. Stream 6 can thus comprise, for example, alkyl aryl carbonate, aromatic alcohol, dialkyl carbonate, aryl alkyl ether and transesterification catalyst. Column 320 can be operated to further drive the reaction toward the desired diaryl carbonate product, while separating other materials for recycle. Two streams can be removed from column 320. The first is a product stream 15, which can be removed from the bottom of column 320, and can comprise essentially all of the diaryl carbonate produced together with residual catalyst, some alkyl aryl carbonate and unwanted high boiling by-products. The product stream 15 can optionally be further distilled and purified in purification section 704 if additional purification is desired.

Stream 16, which can be removed from the distillate of column 320 as a recycle stream, can comprise essentially all of the unreacted aromatic alcohol starting material, and some dialkyl carbonate and by produced alkyl aryl ether, and be recycled to make up part of stream 3.

Column 320 can be operated at a temperature greater than or equal to 100°C, specifically greater than or equal to 120°C, and more specifically greater than or equal to 140°C. Examples of temperature ranges include 100°C to 140°C, specifically 120°C to 250°C, and 140°C to 240°C. The operating pressure in column 320 can be greater than or equal to 10 mbar (1 kPa), specifically greater than or equal to 50 mbar (5 kPa), and more specifically greater than or equal to 100 mbar (10 kPa). Examples of pressure ranges include, 50 mbar to 3 bar (5 kPa to 300 kPa), 50 mbar to 1 bar (5 kPa to 100 kPa), and 200 mbar to 900 mbar (20 kPa to 90 kPa).

Recovering an additional stream (stream 13) from column 210, e.g., such as a side draw, which can then be fed into column 810, can help to reduce the overall number of distillation columns needed to effectively produce diaryl carbonate. For example, stream 13 can comprise dialkyl carbonate, alkyl alcohol (alkanol), by produced alkyl aryl ether and aryl alkyl carbonate, and be fed directly into column 810. A product stream 14 can be recovered from, e.g., the top of 810 and recycled back to 210. For example, stream 14 can comprise mainly dialkyl carbonate, with some alkanol. An aryl alkyl ether rich stream can be recovered from, e.g., the bottom of column 810 through stream 12 and removed from the plant.

Column 810 can be operated at a temperature greater than or equal to 50°C, specifically greater than or equal to 80°C, more specifically greater than or equal to 100°C. Examples of temperature ranges include 100 to 190°C. The operating pressure in column 810 can be greater than or equal to 0.5 bar, specifically greater than or equal to 0.7 bar, and more specifically greater than or equal to I bar. Examples of a pressure range include 1 to 3 bars.

Optionally, stream 15 described above can be sent to flash vessel 510 in which diaryl carbonate, alkyl aryl carbonate and phenol can be vaporized and separated from the catalyst and by produced heavy components (with boiling point higher than diaryl carbonate). Streams 9 and 10 can then be recovered from flash vessel 510. Stream 10 can be recovered from, e.g., the bottom of flash vessel 510 and can comprise catalyst, high boilers and some diaryl carbonate, which can be introduced to evaporator 520. Stream 9, which can comprise diaryl carbonate, alkyl aryl carbonate and aromatic alcohol, can be recovered from top of flash vessel 510 and mixed with stream 11 to form stream 19 and enter a second divided wall distillation column (DWC2) 201, which can be located in the purification section 704 of FIG. 3. Optionally, a second divided wall distillation column (DWC2) 201, which can combine columns 610 and 620 depicted in FIG. 7, can additionally or alternatively employed. As shown in FIG. 7, stream 9, which can comprise diaryl carbonate, alkyl aryl carbonate and aromatic alcohol can be recovered from top of flash vessel 510 and mixed with stream 11 to form stream 19 and enter a first purification column 610. Column 610 recovers a phenyl methyl carbonate rich stream 17 as distillate and DPC rich stream 19' that can be further purified in a second purification column 620. Commercial quality DPC product can be recovered from column 620 as distillate.

Flash vessel 510 in FIG. 3 can be operated at a temperature greater than or equal to 140°C, specifically greater or equal to 150°C, more specifically greater or equal to 160°C. The operating pressure in flash vessel 510 can be greater than or equal to 1 mbar, specifically greater than or equal to 10 mbar, and more specifically greater than or equal to 15 mbar. Examples of pressure ranges include 15 to 150 mbar.

Stream 11 in FIG. 3 can be recovered from, e.g., the top of evaporator 520 and can comprise diaryl carbonate to be mixed with stream 9. Stream 20 can be recovered from, e.g., the bottom of column 520 and split into recycle stream 21 to recycle catalyst back to column 210 by mixing with stream 1, and stream 22 to enter waste station W for processing and disposal as ashes. Evaporator 520 can be operated at a temperature greater than or equal to 150°C, specifically greater or equal to 160°C, more specifically greater or equal to 170°C. The operating pressure in evaporator 520 can be greater than or equal to 1 mbar, specifically greater than or equal to 2 mbar, and more specifically greater than or equal to 5 mbar. Examples of pressure ranges include 5 to 50 mbar.

At least three streams can be recovered from DWC2 (201). For example, stream 17, described above, which can comprise unreacted alkyl aryl carbonate, can be recovered from, e.g., the top of column DWC2 201 and recycled to help form stream 18 which enters the second reactive distillation column 320. Stream 24 can be recovered from, e.g., the side of DWC2 201 and can comprise pure diaryl carbonate, e.g., DPC (impurities just in trace amounts). From, e.g., the bottom of DWC2 201, stream 25 can be obtained and can comprise mainly waste products sent to waste station W for processing and disposal as ashes. The temperature profile of column DWC2 can be greater than or equal to 60°C, specifically greater than or equal to 70°C. Examples of temperature ranges for the temperature profile in the column DWC2 include 60°C to 240°C, specifically 70°C to 210°C. The operating pressure in column DWC2 can be greater than or equal to 1 mbar (100 Pa), specifically greater than or equal to 10 mbar (1 kPa). Examples of pressure include 10 to 50 mbar (1 to 5 kPa). It is noted that the DWC2 can have the structure and operating conditions as described above with respect to DWC1. Optional entry into second purification column 620 from column 610 of FIG. 7 for further purification and recovery of a diaryl carbonate product stream, need not be utilized. The utilization of, e.g., the afore-referenced processing equipment 610 and 620 can be reduced with use of DWC2 201 thereby even further reduces cost and energy consumption.

As in, for example, the afore-described configuration of FIG. 3, the conditions in column 210, such as temperature, pressure and molar ratio of dialkyl carbonate and aromatic alcohol feeds can be selected and optimized to reduce or otherwise control the concentration of, e.g., DMC in the bottom product stream 6. By means of an exemplary strategy, an additional reactive distillation column, such as column B1 described in the afore-referenced U.S. Patent 6,294,684 or column 310 in FIG. 7, distillation of DMC can be avoided because, for example, most of the DPC formation can advantageously take place just in the second reactive distillation column 320 described herein. Column 320 can be directly fed by, e.g., the bottom product stream of column 210 (stream 6) together with a PMC recycle stream (stream 17) from purification section 704 (e.g., DWC2 201 overheads) to form stream 18. Thus, it is not necessary to have an additional column between the flow from reactive distillation column 210 and reactive distillation column 320. By way of contrast, U.S. Patent 6,294,884 discloses an additional column B1. Thus, as a result of significant effort by the inventors, the need for processing equipment, such as the afore-referenced additional distillation column B1, as well as afore-referenced columns 610 and 620 (and C61 and C62), can be avoided while maintaining production rates and quality.

Moreover, further savings in cost as a result of reduced processing equipment and reduced energy consumption can be realized by using the afore-referenced divided wall distillation columns, DWC1 200 and DWC2 201. The use of DWC1 200 and DWC2 201 also provides enhanced flexibility that can result in other advantages. For example, the operating pressures of DWC1 200 can be conveniently adjusted to thermally integrate, directly or indirectly, by using a heat carrier as low pressure steam (LLS), the condenser duty released from column 210 and/or 320 to fulfill the reboiler duty of DWC1 200.

Moreover, an additional advantage obtained by operation of DWC1 200, for example, is the ability to tailor the proportion of, e.g., methanol in the methanol/DMC azeotrope. Different azeotrope compositions can be obtained by increasing or otherwise modifying the pressure profile along the DWC1 200. A DMC rich stream can then be advantageously recovered and recycled to feed column 210 from, e.g., the bottom of DWC1 200.

Also, a further degree of integration between the DMC and DPC processes can be achieved by, e.g., the ability to merge a distillation column from the DPC plant section and a distillation column from the DMC plant section into a single dividing wall distillation column (e.g., DWC1 200). Thus, a single combined feed (e.g., stream 5' of FIG. 3) can be sent to the DWC1 200. Product obtained from, e.g., the bottom of this column (stream 426 of FIG. 3) can be pure DMC (impurities in trace amounts) that can be directly sent to the PMC production column (first reactive distillation column 210). Desirably, no water is present in the DMC product stream (stream 426) as water can negatively affect the catalyst in the reactions in column 210 and 320. Product from, e.g., the top of DWC1 200 (stream 431) can comprise mainly a mixture of methanol and dimethyl carbonate azeotrope concentration. Stream 416 from, e.g., a middle height side draw can be a mixture of DMC/water and traces of methanol that can be sent to decanter 610 where two phases can be obtained at, e.g., ambient temperature. An organic phase of, e.g., mostly DMC, can be returned to DWC1 200 via stream 418, while an aqueous phase can be sent to waste water recovery column 630 via stream 421. Pure water (impurities in trace amounts) can then be obtained from, e.g., the bottom of column 630 from stream 422, and stream 424 comprising, e.g., azeotrope DMC/water can be produced from, e.g., the overheads and recycled back to decanter 610. Thus, the introduction of DWC1 200 not only merges two columns, but can also avoid the need for an additional DMC recovery column, and can reduce energy consumption throughout the system.

Moreover, with further reference to FIG. 3, feed to column 810 from stream 13 can be taken as a side draw from column 210. Conditions in the rectifying section of column 210 can be adjusted to obtain a composition profile with a peak in concentration of anisole to be purged from the system and a negligible concentration of phenol at the stage of extraction.

Advantageously, a DPC rich stream (stream 19) can be sent to purifying DWC2 201, which can be used to recover unreacted PMC and phenol from, e.g., overhead stream 17 and recover pure DPC (impurities in trace amounts) as of stream 24.

In an embodiment, a method for production of diaryl carbonate by reaction of a dialkyl carbonate and an aromatic alcohol in the presence of a transesterification catalyst, comprising: producing a first top stream (5) comprising dialkyl carbonate and alkyl alcohol from a first reactive distillation column (210); introducing a reactant stream (5') comprising the first top stream (5) to a first divided wall distillation column (DWC1 200) and separating the mixture with use of the first divided wall distillation column (DWC1 200); recovering from the first reactive distillation column (210) a first bottom stream (6) comprising alkyl aryl carbonate; and introducing the first bottom stream (6) into a second reactive distillation column (320) to produce a second bottom stream (15) comprising diaryl carbonate.

In the various embodiments: (i) the method further comprises recovering from the second reactive distillation column (320) a second bottom stream (15) comprising diaryl carbonate and alkyl aryl carbonate, and a second top stream (16) comprising aromatic alcohol and dialkyl carbonate, and recycling the second top stream (16) to the first reactive distillation column (210), recovering from the first rectification column (810) a third bottom stream (12) comprising alkyl aryl ether and a third top stream (14) comprising dialkyl carbonate and alkyl alcohol, and recycling the third top stream (14) to the first reactive distillation column (210), recovering from the first divided wall distillation column (DWC1 200) an azeotrope fourth top stream (431) and recycling the azeotrope fourth top stream (431) to the crude product separation column (160), and recovering a dialkyl carbonate recycle stream/fourth bottom stream (426) from the first divided wall distillation column (DWC1 200) and recycling the dialkyl carbonate recycle stream (426) to the first reactive distillation column (210); and/or (ii) the method further comprises introducing the second bottom stream (15) to a flash vessel (510), recovering from the flash vessel (510) a fifth bottom stream (10) comprising catalyst and diaryl carbonate and a fifth top stream (9) comprising diaryl carbonate, alkyl aryl carbonate and aromatic alcohol, introducing the fifth bottom stream (10) to an evaporator (520), and recovering from the evaporator (520) a sixth top stream (11) comprising diaryl carbonate and a sixth bottom stream (20) comprising the catalyst and diaryl carbonate, and recycling the catalyst to first reactive distillation column (210); and/or (iii) the method further comprises introducing the sixth top stream (11) to a second divided wall distillation column (DWC2 201), recovering from the second divided wall distillation column (DWC2 201) a seventh top stream (17) comprising unreacted aryl alkyl carbonate and aromatic alcohol, and recycling the seventh top stream (17) to the second reactive distillation column (320), and recovering from the second divided wall distillation column (DWC2 201) a product stream (24) comprising diaryl carbonate; and/or (iv) the method further comprises recovering a second stream (416) comprising dialkyl carbonate, water and alkanol from the first divided wall distillation column (DWC1 200) and introducing the second stream (416) into a decanter (610), and recovering from the decanter (610) a third stream (418) comprising dialkyl carbonate and recycling the third stream (418) to the first divided wall distillation column (DWC1 200); and/or (v) the method further comprises recovering from the decanter (610) a fourth stream (421) comprising water, alkanol and dialkyl carbonate, introducing the fourth stream (421) into a water recovery distillation column (630), recovering from the water recovery distillation column (630) a fifth stream (424) comprising water, alkanol and dialkyl carbonate, and a sixth steam (422) comprising water; and recycling the fifth stream (424) to the decanter (610); and/or (vi) the method further comprises wherein the dialkyl carbonate comprises dimethyl carbonate, the alkyl alcohol comprises methanol, the dialkyl carbonate recycle stream (426) comprises purified dimethyl carbonate with impurities in trace amounts, the aromatic alcohol comprises phenol and the alkyl aryl carbonate comprises methyl phenyl carbonate; and/or (vii) the first reactive distillation column (210) is maintained at a temperature of 120°C to 250°C, and a pressure at a top of the first reactive distillation column (210) of 4 to 6 bars (400 to 600 kPa); and/or (viii) the second reactive distillation column (320) is maintained at a temperature of 140°C to 240°C, and a pressure at a top of the second reactive distillation column (320) of 0.2 to 0.9 bars (20 to 90 kPa); and/or (ix) the first rectification column (810) is maintained at a temperature of 100°C to 190°C, and a pressure at a top of the column (810) of 1 to 3 bars (100 to 300 kPa); and/or (x) the first divided wall distillation column (DWC1 200) is maintained at a temperature of 60°C to 140°C, and a pressure at a top of the column (200) of 1.2 to 3 bars (120 to 300 kPa); and/or (xi) the second divided wall distillation column (DWC2 201) is maintained at a temperature of 70°C to 210°C, and a pressure at a top of the column (201) of 10 to 50 mbar (1 to 5 kPa); and/or (xii) the reactant stream (5') further comprises an effluent reactant stream (412), which exits a crude product separation column (160) in a dialkyl carbonate production unit and which comprises dialkyl carbonate, alkyl alcohol and water; and/or (xiii) the method further comprises recovering from the first reactive distillation column (210) a first side stream (13) comprising dialkyl carbonate, and introducing the first side stream (13) to a first rectification column (810); and/or (xiv) the method further comprises introducing the second bottom stream (15) to a purification section (704) without passing through another reactive distillation column; and/or (xv) the dialkyl carbonate comprises dimethyl carbonate; and/or (xvi) the diaryl carbonate comprises diphenyl carbonate; and/or (xvii) the aromatic alcohol comprises phenol; and/or (xviii) the alkyl aryl carbonate comprises methyl phenyl carbonate; and/or (xix) the alkyl alcohol comprises methanol; and/or (xx) the dialkyl carbonate is dimethyl carbonate, the diaryl carbonate is diphenyl carbonate, the aromatic alcohol is phenol, the alkyl aryl carbonate is methyl phenyl carbonate, and the alkyl alcohol is methanol.

In an embodiment, an apparatus for continuous production of diaryl carbonate by reaction of a dialkyl carbonate and an aromatic alcohol in the presence of a transesterification catalyst, can comprise: a first reactive distillation column (210), a second reactive distillation column (320), a first rectification column (810), a first divided wall distillation column (DWC1 200) and a plurality of lines for transporting reactant and product streams. The first reactive distillation column (210) can be connected to input lines for introduction of reactants, and to a first transfer line, a second transfer line and a third transfer line, the first transfer line extends from the top of the first reactive distillation column (210) to an inlet of the first divided wall distillation column (DWC1 200), the second transfer line extends from the bottom of first reactive distillation column (210) to an inlet of the second reactive distillation column (320) without passing through another reactive distillation column, and the third transfer line extends from the side of the first reactive distillation column (210) to an inlet of the first rectification column (810). The second reactive distillation column (320) can be connected to a first recycle line extends from the top of the second reactive distillation column (320) to an inlet of the first reactive distillation column (210), and a first product line extends from the bottom of the second reactive distillation column (320) for providing diaryl carbonate. The first rectification column (810) can be connected to a fourth transfer line, the fourth transfer line extends from the top of the first rectification column (810) to an inlet of the first reactive distillation column (210). The first divided wall distillation column (DWC1 200) can be connected to a second product line for providing dialkyl carbonate/alkyl alcohol azeotrope extends from the top of the first divided wall distillation column (DWC1 200) through a crude product separation column (160) to an inlet of the first reactive distillation column (210), and a second recycle line extends from the bottom of the first divided wall distillation column (DWC1 200) to an inlet of the first reactive distillation column (210).

In the various embodiments of the apparatus: (i) the apparatus further comprises a flash vessel (510), an evaporator (520), and a second divided wall distillation column (DWC2 201), wherein the flash vessel (510) is connected to a fifth line and a sixth line, the fifth line extends from the top of the flash vessel (510) to an inlet of the second divided wall distillation column (DWC2 201), and the sixth line extends from the bottom of the flash vessel (510) to an inlet of the evaporator (520); and/or (ii) the evaporator (520) is connected to a seventh line and a third recycle line, the seventh line extends from the top of the evaporator (520) to an inlet of the second divided wall distillation column (DWC2 201) and the third recycle line extends from the bottom of the evaporator (520) to an inlet of the first reactive distillation column (210); and/or (iii) the dialkyl carbonate comprises dimethyl carbonate; and/or (iv) the diaryl carbonate comprises diphenyl carbonate; and/or (v) the aromatic alcohol comprises phenol; and/or (vi) the alkyl aryl carbonate comprises methyl phenyl carbonate; and/or (vii) the alkyl alcohol comprises methanol; and/or (viii) the dialkyl carbonate is dimethyl carbonate, the diaryl carbonate is diphenyl carbonate, the aromatic alcohol is phenol, the alkyl aryl carbonate is methyl phenyl carbonate, and the alkyl alcohol is methanol.

The process and apparatus described above, in accordance with embodiments, are further illustrated by the following non-limiting Example, which are based, in part, on computer simulation for a proposed production facility (e.g., FIG. 3).

### EXAMPLE

In this example and with reference to FIG. 3 described above, stream 412 comprising 35,389.8 kilogram/hour (kg/h) (48.7 wt% MeOH, 45.0 wt% DMC, 5.3 wt% water, 0.6 wt% methylal, 0.2 wt% dimethyl ether and 0.1 wt% methyl chloride) obtained from the top of column 160 of an exemplary DMC plant was mixed with (stream 5) 44,679.3 kg/h (89.3 wt% DMC and 10.7 wt% MeOH) obtained from the top of PMC production column 210.

This mixed stream forming stream 5' was fed to DWC1 200, operating close to atmospheric pressure, specifically operating at 1.45 bar (145 kPa). Dividing wall distillation column DWC1 200 comprised the following sections: a) an inflow section and b) an offtake section, both with forty trays and separated by a vertical wall, c) an upper column section of twenty trays and d) a lower column section of twenty trays, e) a condenser and f) a reboiler.

Overhead flow rate (stream 431) from the DWC1 200 was 31,706.5 kg/h with a composition of 69.9 wt% MeOH, 29.0 wt% DMC, 20 ppm water, 0.7 wt% methylal, 0.2 wt% dimethyl ether and 0.2 wt% methyl chloride. A side-draw (stream 416) of 16,606.5 kg/h was taken from the offtake section of the DWC1 200 at the 17^{th} stage of this section (counting from bottom to top) with a composition of 86.2 wt% DMC, 13.8 wt% water and 13 ppm MeOH. Stream 416 was decanted in decanter 610 and split in two phases, an organic phase (stream 418) and an aqueous phase (stream 421). In particular, 14,705.9 kg/h of organic phase in stream 418 (97.4 wt% DMC, 2.6 wt% water and 15 ppm MeOH) was returned back to the top stage of the lower section of DWC1 200. Regarding the aqueous phase, 2,302.7 kg/h (87.0 wt% water, 13.0 wt% DMC and 95 ppm MeOH) was sent to distillation column 630 (waste water recovery column). Column 630 purged 1,900.6 kg/h of 100 wt% water as a bottom residue, while 402.1 kg/h of 74.4 wt% DMC, 25.6 wt% water and 539 ppm MeOH was recycled back to the decanter 610 in stream 424. From stream 426, 46,698.0 kg/h of pure DMC (impurities in trace amounts) was obtained from the bottom of DWC1 200. Stream 426 was recycled and fed to the PMC production column 210.

It is further noted that 13,998.0 kg/h of fresh phenol (stream 1) was mixed with a phenolic recycle stream (stream 16) of 38,327.7 kg/h (72.8 wt% PhOH, 26.9 wt% DMC, 0.1 wt % MeOH, 0.01 wt% anisole) and preheated to 147°C. From stream 21, 2,042.9 kg/h of recycled catalyst (66.3 wt% titanium tetraphenoxide catalyst, 20.5 wt% DPC, 12.8 wt% by-produced high-boilers (e.g., having a boiling point that is greater than DPC (excluding the catalyst; such as xanthone, phenyl 2-methoxy bentoate, etc.) recovered from the purification section 704 of the process were merged with the fresh phenol stream (stream 1) and fed at a middle height to the first reactive distillation column 210. In order to compensate for catalyst losses from the purge, 128 kg/h of fresh catalyst (34.3 wt% titanium tetraphenoxide catalyst, 65.7 wt% phenol) was continuously fed to the catalyst recycle loop through stream 0.

Column 210 included twenty distillation trays in the rectifying section and twenty-four reactive trays with a hold-up of 0.7 cubic meters (m³) in the stripping section. A kettle-type reboiler with a hold-up of 5m³ supplied the heating of the column. The first reactive distillation column 210 also was equipped with a condenser. Temperature and pressure profiles were, respectively, 241°C and 5.1 bars (510 kPa), in the reboiler, and 127°C and 4.7 bars (470 kPa) at the top of the column. The reflux ratio of the column was 1.55.

The bottom rate (stream 6) from 210 was 56,307.8 kg/h with a composition of 49.6 wt% phenol, 32.6 wt% PMC, 8.7 wt% DMC, 6.0 wt% DPC, 2.5 wt% titanium tetraphenoxide catalyst, 0.5 wt% HBs, 0.06 wt% MeOH and 0.04 wt% anisole. A side-draw (stream 13) of 4,000.1 kg/h (91.3 wt% DMC, 7.2 wt% anisole, 1.5 wt% MeOH, 0.01 wt% PhOH) was taken from the liquid phase of the seventh stage of the rectifying section (counting from top). This lateral draw stream (stream 13) was fed to the top stage of column 810 that included nine stripping stages, a condenser and a reboiler. An anisole rich stream (stream 12) of 138 kg/h (99.9 wt% alkyl aryl ether (anisole), 0.01 wt% PhOH and 0.1 wt% DMC) was obtained as bottom product and discarded, while 3,862 kg/h (94.6 wt% DMC, 3.8 wt% anisole and 1.6 wt% MeOH) was obtained as distillate from column 810 and recycled back to the seventh stage of column 210 by stream 14.

Bottom product from 210 (stream 6) and 3,952.3kg/h of recycled PMC (stream 17) (94.6 wt% PMC, 4.2 wt% PhOH and 1.2 wt% DMC) was fed to column 320 in whose bottom section the PMC disproportionation reaction takes place. The phenol-rich stream (stream 16) obtained from its overheads was recycled to column 210, while 21,932.5 kg/h (74.3 wt% DPC, 17.0 wt% PMC, 0.2 wt% DMC, 6.4 wt% titanium tetraphenoxide catalyst and 1.2 wt% HBs) was sent to flash vessel 510 by stream 15. In the feed vaporizer vessel (flash vessel) 510, 16,451.7 kg/h (76.4 wt% DPC, 22.3 wt% PMC, 1.0 wt% phenol and 0.3 wt% DMC) was vaporized and separated from a heavy stream of 5,480.8 kg/h of DPC, titanium tetraphenoxide catalyst and a series of high boiling reaction by-products (HBs).

Heavy stream (stream 10) from the bottom product of 510, including 68 wt% DPC, was further treated in 520, which recovered 3,374.5 kg/h of DPC (stream 9). From the bottom of 520, 2,106.3 kg/h (stream 20) was recycled back to the PMC production column (210) through stream 21, wherein 63.4 kg/h of stream 20 was purged and sent to the incinerator W by stream 22. Recovered DPC from the falling thin-film evaporator (520) was condensed and fed to the DPC dividing wall distillation/purification column (DWC2 201). DWC2 201 comprised the following sections: a) an inflow section and b) an off-take section, both with sixteen trays and separated by a vertical wall, c) an upper column section of eight trays and d) a lower column section of eight trays, e) a condenser and f) a reboiler.

DWC2 201 distilled the unreacted PMC, which was recycled to 320 by stream 17, while its bottom product was a stream (stream 25) of 96.6 kg/h, which was purged and sent to an incinerator W. A side-draw (stream 24) of 15,777 kg/h of pure DPC (impurities in trace amounts) was taken from the off-take section of the DWC2 201 at the fourth stage of this section (counting from bottom to top).

In addition to the reduction of piping and process equipment, embodiments disclosed herein can save, for example, greater than or equal to 19% of medium-pressure and greater than or equal to 20% of high-pressure steams compared to other designs having the otherwise same equipment and flow rates. For example, pure DMC product (impurities in trace amounts) can be produced using a divided wall distillation column, which can reduce the number of columns needed, and thus reduce energy consumption and overall cost of production. For example, DMC 1 200 can replace C410 as well as columns 301 and 302, (as compared to the same system otherwise (e.g., no additional equipment or change in the size of the other equipment or the flow rates, to compensate for the replacement).

In general, embodiments may alternately comprise (e.g., include), consist of, or consist essentially of, any appropriate components herein disclosed. The embodiments may additionally, or alternatively, be formulated so as to be devoid, or substantially free, of any components, materials, ingredients, adjuvants or species used in the prior art compositions or that are otherwise not necessary to the achievement of the function and/or objectives of the embodiments.

As used herein, a trace amount is an amount of less than 0.01 wt% based upon a total weight of the product. All ranges disclosed herein are inclusive of the endpoints, and the endpoints are independently combinable with each other (e.g., ranges of "less than or equal to 25 wt%, or, more specifically, 5 wt% to 20 wt%", is inclusive of the endpoints and all intermediate values of the ranges of "5 wt% to 25 wt%," etc.). "Combination" is inclusive of blends, mixtures, alloys, reaction products, and the like. Furthermore, the terms "first," "second," and the like, herein do not denote any order, quantity, or importance, but rather are used to distinguish one element from another. The terms "a" and "an" and "the" herein do not denote a limitation of quantity, and are to be construed to cover both the singular and the plural, unless otherwise indicated herein or clearly contradicted by context. The suffix "(s)" as used herein is intended to include both the singular and the plural of the term that it modifies, thereby including one or more of that term (e.g., the film(s) includes one or more films). Reference throughout the specification to "one embodiment", "another embodiment", "an embodiment", and so forth, means that a particular element (e.g., feature, structure, and/or characteristic) described in connection with the embodiment is included in at least one embodiment described herein, and may or may not be present in other embodiments. In addition, it is to be understood that the described elements may be combined in any suitable manner in the various embodiments.

## Claims

1. A method for production of diaryl carbonate by reaction of a dialkyl carbonate and an aromatic alcohol in the presence of a transesterification catalyst, comprising:
producing a first top stream (5) comprising dialkyl carbonate and alkyl alcohol from a first reactive distillation column (210);
introducing a reactant stream (5') comprising the first top stream (5) to a first divided wall distillation column (DWC1 200) and separating the mixture with use of the first divided wall distillation column (DWC1 200);
recovering from the first reactive distillation column (210) a first bottom stream (6) comprising alkyl aryl carbonate; and
introducing the first bottom stream (6) into a second reactive distillation column (320) to produce a second bottom stream (15) comprising diaryl carbonate;
recovering from the second reactive distillation column (320) the second bottom stream (15) comprising diaryl carbonate and alkyl aryl carbonate, and a second top stream (16) comprising aromatic alcohol and dialkyl carbonate, and recycling the second top stream (16) to the first reactive distillation column (210);
recovering from the first reactive distillation column (210) a first side stream (13) comprising dialkyl carbonate, and introducing the first side stream (13) to a first rectification column (810);
recovering from the first rectification column (810) a third bottom stream (12) comprising alkyl aryl ether and a third top stream (14) comprising dialkyl carbonate and alkyl alcohol, and recycling the third top stream (14) to the first reactive distillation column (210);
recovering from the first divided wall distillation column (DWC1 200) an azeotrope fourth top stream (431) and recycling the azeotrope fourth top stream (431) to the crude product separation column (160); and
recovering a dialkyl carbonate recycle stream/fourth bottom stream (426) comprising a purified dialkyl carbonate from the first divided wall distillation column (DWC1 200) and recycling the dialkyl carbonate recycle stream (426) to the first reactive distillation column (210);
recovering a second stream (416) comprising dialkyl carbonate, water and alkanol from a side of the first divided wall distillation column (DWC1 200) and introducing the second stream (416) into a decanter (610); and
recovering from the decanter (610) a third stream (418) comprising dialkyl carbonate and recycling the third stream (418) to the first divided wall distillation column (DWC1 200).

2. The method of Claim 1, further comprising
introducing the second bottom stream (15) to a flash vessel (510);
recovering from the flash vessel (510) a fifth bottom stream (10) comprising catalyst and diaryl carbonate and a fifth top stream (9) comprising diaryl carbonate, alkyl aryl carbonate and aromatic alcohol;
introducing the fifth bottom stream (10) to an evaporator (520); and
recovering from the evaporator (520) a sixth top stream (11) comprising diaryl carbonate and a sixth bottom stream (20) comprising the catalyst and diaryl carbonate, and recycling the catalyst to first reactive distillation column (210).

3. The method of Claim 2, further comprising:
introducing the sixth top stream (11) to a second divided wall distillation column (DWC2 201);
recovering from the second divided wall distillation column (DWC2 201) a seventh top stream (17) comprising unreacted aryl alkyl carbonate and aromatic alcohol, and recycling the seventh top stream (17) to the second reactive distillation column (320); and
recovering from the second divided wall distillation column (DWC2 201) a product stream (24) comprising diaryl carbonate.

4. The method of Claim 1, comprising:
recovering from the decanter (610) a fourth stream (421) comprising water, alkanol and dialkyl carbonate;
introducing the fourth stream (421) into a water recovery distillation column (630);
recovering from the water recovery distillation column (630) a fifth stream (424) comprising water, alkanol and dialkyl carbonate, and a sixth steam (422) comprising water; and recycling the fifth stream (424) to the decanter (610).

5. The method of any of Claims 1-4, wherein the dialkyl carbonate comprises dimethyl carbonate, the alkyl alcohol comprises methanol, the dialkyl carbonate recycle stream (426) comprises purified dimethyl carbonate with impurities in trace amounts, the aromatic alcohol comprises phenol and the alkyl aryl carbonate comprises methyl phenyl carbonate.

6. The method of any of Claims 1-5, wherein the first reactive distillation column (210) is maintained at a temperature of 120°C to 250°C, and a pressure at a top of the first reactive distillation column (210) of 4 to 6 bars (400 to 600 kPa).

7. The method of any of Claims 1-6, wherein the second reactive distillation column (320) is maintained at a temperature of 140°C to 240°C, and a pressure at a top of the second reactive distillation column (320) of 0.2 to 0.9 bars (20 to 90 kPa).

8. The method of any of Claims 1-7, wherein the first rectification column (810) is maintained at a temperature of 100°C to 190°C, and a pressure at a top of the column (810) of 1 to 3 bars (100 to 300 kPa).

9. The method of any of Claims 1-8, wherein the first divided wall distillation column (DWC1 200) is maintained at a temperature of 60°C to 140°C, and a pressure at a top of the first divided wall distillation column (DWC1 200) of 1.2 to 3 bars (120 to 300 kPa).

10. The method of Claim 3, wherein the second divided wall distillation column (DWC2 201) is maintained at a temperature of 70°C to 210°C, and a pressure at a top of the second divided wall distillation column (DWC2 201) of 10 to 50 mbar (1 to 5 kPa).

11. The method of any of Claims 1-10, wherein the reactant stream (5') further comprises an effluent reactant stream (412) directed from a reaction section (420) of a dialkyl carbonate production facility, which exits a crude product separation column (160) in a dialkyl carbonate production unit and which comprises dialkyl carbonate, alkyl alcohol and water.

12. The method of any of Claims 1-11, further comprising introducing the second bottom stream (15) to a purification section (704) without passing through another reactive distillation column.

13. An apparatus for continuous production of diaryl carbonate by reaction of a dialkyl carbonate and an aromatic alcohol in the presence of a transesterification catalyst, the apparatus comprising: a first reactive distillation column (210), a second reactive distillation column (320), a first rectification column (810), a first divided wall distillation column (DWC1 200) and a plurality of lines for transporting reactant and product streams;
wherein the first reactive distillation column (210) is connected to input lines for introduction of reactants, and to a first transfer line, a second transfer line, and a third transfer line, the first transfer line extends from the top of the first reactive distillation column (210) to an inlet of the first divided wall distillation column (DWC1 200), the second transfer line extends from the bottom of first reactive distillation column (210) to an inlet of the second reactive distillation column (320) without passing through another reactive distillation column, and the third transfer line extends from the side of the first reactive distillation column (210) to an inlet of the first rectification column (810);
wherein the second reactive distillation column (320) is connected to a first recycle line extends from the top of the second reactive distillation column (320) to an inlet of the first reactive distillation column (210), and a first product line extends from the bottom of the second reactive distillation column (320) for providing diaryl carbonate;
wherein the first rectification column (810) is connected to a fourth transfer line, the fourth transfer line extends from the top of the first rectification column (810) to an inlet of the first reactive distillation column (210); and
wherein the first divided wall distillation column (DWC1 200) is connected to a second product line for providing dialkyl carbonate/alkyl alcohol azeotrope extends from the top of the first divided wall distillation column (DWC1 200) through a crude product separation column (160) to an inlet of the first reactive distillation column (210), a middle height side draw line extends from a middle of the first divided wall distillation column (DWC1 200) to a decanter (610), a third line that extends from the decanter (610) to the first divided wall distillation column (DWC1 200), and a second recycle line extends from the bottom of the first divided wall distillation column (DWC1 200) to an inlet of the first reactive distillation column (210).

14. The apparatus of Claim 13, further comprising a flash vessel (510), an evaporator (520), and a second divided wall distillation column (DWC2 201), wherein:
the first product line extends to the flash vessel (510), which is connected to a fifth line and a sixth line, the fifth line extends from the top of the flash vessel (510) to an inlet of the second divided wall distillation column (DWC2 201), and the sixth line extends from the bottom of the flash vessel (510) to an inlet of the evaporator (520).

15. The apparatus of Claim 14, wherein the evaporator (520) is connected to a seventh line and a third recycle line, the seventh line extends from the top of the evaporator (520) to an inlet of the second divided wall distillation column (DWC2 201) and the third recycle line extends from the bottom of the evaporator (520) to an inlet of the first reactive distillation column (210).

16. The apparatus of any of Claims 13-15, wherein the dialkyl carbonate comprises dimethyl carbonate, the diaryl carbonate comprises diphenyl carbonate, the aromatic alcohol comprises phenol, and the alkyl alcohol comprises methanol.

## Patentansprüche

1. Verfahren zur durchgehenden Herstellung von Diarylcarbonat durch Umsetzung eines Dialkylcarbonats und eines aromatischen Alkohols in Gegenwart eines Transesterifizierungskatalysators, umfassend:
Herstellen eines ersten oberen Stroms (5), umfassend Dialkylcarbonat und Alkylalkohol aus einer ersten reaktiven Destillationssäule (210);
Einleiten eines ersten Reaktantstroms (5'), umfassend den ersten oberen Strom (5), zu einer ersten Trennwand-Destillationssäule (DWC1 200) und Abscheiden der Mischung mithilfe der ersten Trennwand-Destillationssäule (DWC1 200);
Rückgewinnen, aus der ersten reaktiven Destillationssäule (210) eines ersten unteren Stromes (6), umfassend Alkylarylcarbonat, und
einleiten des ersten unteren Stroms (6) in eine zweite reaktive Destillationssäule (320) zum Erzeugen eines zweiten unteren Stroms (15), umfassend Diarylcarbonat;
Rückgewinnen, von der zweiten reaktiven Destillationssäule (320), des zweiten unteren Stroms (15), umfassend Diarylcarbonat und Alkylarylcarbonat, und eines zweiten oberen Stroms (16), umfassend aromatischen Alkohol und Dialkylcarbonat, und Rückführen des zweiten oberen Stroms (16) zur ersten reaktiven Destillationssäule (210);
Rückgewinnen, von der ersten reaktiven Destillationssäule (210) eines ersten Seitenstroms (13), umfassend Dialkylcarbonat, und Einleiten des ersten Seitenstroms (13) zu einer ersten Rektifiziersäule (810);
Rückgewinnen, aus der ersten Rektifiziersäule (810), eines dritten unteren Stroms (12), umfassend Alkylarylether und eines dritten oberen Stroms (14), umfassend Dialkylcarbonat und Alkylalkohol, und wahlweise Rückführen des dritten oberen Stroms (14) in die erste reaktive Destillationssäule (210);
Rückgewinnen, von der ersten Trennwand-Destlillationssäule (DWC1 200) eines azeotropen vierten oberen Stroms (431) und Rückführen des azeotropen vierten oberen Stroms (431) zu der Rohproduktaustragssäule (160); und
Rückgewinnen eines Dialkylcarbonat-Rückführstroms/vierten unteren Stroms (426), umfassend aufgereinigtes Dialkylcarbonat von der ersten Trennwand-Destillationssäule (DWC1 200) und Rückführen des Dialkylcarbonat-Rückführstroms (426) zur ersten reaktiven Destillationssäule (210);
Rückgewinnen eines zweiten Stroms (416), umfassend Dialkylcarbonat, Wasser und Alkanol von einer Seite der ersten Trennwand-Destillationssäule (DWC1 200) und Einleiten des zweiten Stroms (416) in einen Dekanter (610); und
Rückgewinnen, von dem Dekanter (610), eines dritten Stroms (418), umfassend Dialkylcarbonat und Rückführen des dritten Stroms (418) zur ersten Trennwand-Destillationssäule (DWC1 200).

2. Verfahren nach Anspruch 1, ferner umfassend:
Einleiten des zweiten unteren Stroms (15) in einen Flash-Behälter (510);
Rückgewinnen, aus dem Flash-Behälter (510) eines fünften unteren Stroms (10), umfassend einen Katalysator und Diarylcarbonat und eines fünften oberen Stroms (9), umfassend Diarylcarbonat, Alkylarylcarbonat und aromatischen Alkohol;
Einleiten des fünften unteren Stroms (10) in einen Verdampfer (520); und
Rückgewinnen, aus dem Verdampfer (520), eines sechsten oberen Stroms (11), umfassend Diarylcarbonat und einen sechsten unteren Strom (20), umfassend den Katalysator und Diarylcarbonat, und Wiederherstellen des Katalysators für die erste reaktive Destillationssäule (210);

3. Verfahren nach Anspruch 2, ferner umfassend:
Einleiten des sechsten oberen Stroms (11) in eine zweite Trennwand-Destillationssäule (DWC2 201);
Rückgewinnen, von der zweiten Trennwand-Destillationssäule (DWC2 201) eines siebten oberen Stroms (17), umfassend nicht umgesetztes Arylalkylcarbonat und einen aromatischen Alkohol, und Rückführen des siebten oberen Stroms (17) in die zweite reaktive Destillationssäule (320); und
Rückgewinnen, von der zweiten Trennwand-Destillationssäule (DWC2 201) eines Produktstroms (24), der Diarylcarbonat umfasst.

4. Verfahren nach Anspruch 1, umfassend:
Rückgewinnen, von dem Dekanter (610) eines vierten Stroms (421), umfassend Wasser, Alkohol und Dialkylcarbonat;
Einleiten des vierten Stroms (421) in eine Wasserrückgewinnungsdestillationssäule (630);
Rückgewinnen, von der Wasserrückgewinnungsdestillationssäule (630) eines fünften Stroms (424), umfassend Wasser, Alkanol und Dialkylcarbonat, und eines sechsten Stroms (422), umfassend Wasser; und Rückführen des fünften Stroms (424) zum Dekanter (610).

5. Verfahren nach einem der Ansprüche 1 bis 4, wobei das Dialkylcarbonat Dimethylcarbonat umfasst, der Alkylalkohol Methanol umfasst, der Dialkylcarbonat-Rückführstrom (426) aufgereinigtes Dimethylcarbonat mit Verunreinigungen in Spurenmengen umfasst, wobei der aromatische Alkohol Phenol und das Alkylarylcarobnat Methylphenylcarbonat umfasst.

6. Verfahren nach einem der Ansprüche 1 bis 5, wobei die erste reaktive Destillationssäule (210) bei einer Temperatur von 120°C bis 250°C gehalten wird, und einem Druck an der Oberseite der ersten reaktiven Destillationssäule (210) von 4 bis 6 bar (400 bis 600 kPa).

7. Verfahren nach einem der Ansprüche 1 bis 6, wobei die zweite reaktive Destillationssäule (320) bei einer Temperatur von 140°C bis 240°C gehalten wird, und einem Druck an der Oberseite der zweiten reaktiven Destillationssäule (320) von 0,2 bis 0,9 bar (20 bis 90 kPa).

8. Verfahren nach einem der Ansprüche 1 bis 7, wobei die erste Rektifiziersäule (810) bei einer Temperatur von 100° C bis 190° C und einem Druck an einer Oberseite der Säule (810) von 1 bis 3 bar (100 bis 300 kPa) gehalten wird.

9. Verfahren nach einem der Ansprüche 1 bis 8, wobei die erste Trennwand-Destillationssäule (DWC1 200) bei einer Temperatur von 60°C bis 140°C gehalten wird, und einem Druck an der Oberseite der ersten Trennwand-Destillationssäule (DWC1 200) von 1,2 bis 3 bar (120 bis 300 kPa).

10. Verfahren nach Anspruch 3, wobei die zweite Trennwand-Destillationssäule (DWC2 201) bei einer Temperatur von 70° C bis 210° C gehalten wird, und einem Druck an der Oberseite der zweiten Trennwand-Destillationssäule (DWC2 201) von 10 bis 50 mbar (1 bis 5 kPa).

11. Verfahren nach einem der Ansprüche 1 bis 10, wobei der Reaktantstrom (5') ferner einen Reaktantabstrom (412) umfasst, der von einem Reaktionsabschnitt (420) einer Dialkylcarbonat-Produktionsanlage gerichtet ist, der aus einer Rohprodukttrennsäule (160) in einer Dialkylcarbonat-Produktionseinheit austritt und Dialkylcarbonat, Alkylalkohol und Wasser umfasst.

12. Verfahren nach einem der Ansprüche 1 bis 11, ferner umfassend das Einleiten des zweiten unteren Stroms (15) zu einem Reinigungsabschnitt (704) ohne Durchlaufen einer weiteren reaktiven Destillationssäule.

13. Vorrichtung zur durchgehenden Herstellung von Diarylcarbonat durch Umsetzung eines Dialkylcarbonats und eines aromatischen Alkohols in Gegenwart eines Transesterifizierungskatalysators, wobei die Vorrichtung umfasst: eine erste reaktive Destillationssäule (210), eine zweite reaktive Destillationssäule (320), eine erste Rektifiziersäule (810), eine erste Trennwand-Destillationssäule (DWC1 200) und eine Mehrzahl von Leitungen zum Transportieren von Reaktant- und Produktströmen;
wobei die erste reaktive Destillationssäule (210) mit Eingangsleitungen zur Einleitung von Reaktanten verbunden ist, und mit einer ersten Transferleitung, einer zweiten Transferleitung und einer dritten Transferleitung, wobei sich die erste Transferleitung von der Oberseite der ersten reaktiven Destillationssäule (210) zu einem Einlass der ersten Trennwand-Destillationssäule (DWC1 200) erstreckt, sich die zweite Transferleitung von der Unterseite der ersten reaktiven Destillationssäule (210) zu einem Einlass der zweiten reaktiven Destillationssäule (320) erstreckt, ohne eine weitere reaktive Destillationsäule zu durchlaufen und sich die dritte Transferleitung von der Seite der ersten reaktiven Destillationssäule (210) zu einem Einlass der ersten Rektifiziersäule (810) erstreckt;
wobei die zweite reaktive Destillationssäule (320) mit einer ersten Rückführleitung verbunden ist, die sich von der Oberseite der zweiten reaktiven Destillationssäule (320) zu einem Einlass der ersten reaktiven Destillationssäule (210) erstreckt, und sich eine erste Produktleitung von der Unterseite der zweiten reaktiven Destillationssäule (320) zum Bereitstellen von Diarylcarbonat erstreckt;
wobei die erste Rektifiziersäule (810) mit einer vierten Transferleitung verbunden ist, sich die vierte Transferleitung von der Oberseite der ersten Rektifiziersäule (810) zu einem Einlass der ersten reaktiven Destillationssäule (210) erstreckt, und
wobei sich die erste Trennwand-Destillationssäule (DWC1 200) mit einer zweiten Produktleitung zum Bereitstellen von Dialkylcarbonat/Alkylalkoholazeotrop von der Oberseite der ersten Trennwand-Destillationssäule (DWC1 200) durch eine Rohprodukttrennsäule (160) zu einem Einlass der ersten reaktiven Destillationssäule (210) erstreckt, sich eine mittelhohe Seitenansaugleitung von einer Mitte der ersten Trennwand-Destillationssäule (DWC1 200) zu einem Dekanter (610) erstreckt, sich eine dritte Leitung von dem Dekanter (610) zu der ersten Trennwand-Destillationssäule (DWC1 200) erstreckt und sich eine zweite Rückführleitung von der Unterseite der ersten Trennwand-Destillationssäule (DWC1 200) zu einem Einlass der ersten reaktiven Destillationssäule (210) erstreckt.

14. Vorrichtung nach Anspruch 13, ferner umfassend einen Flash-Behälter (510), einen Verdampfer (520) und eine zweite Trennwand-Destillationssäule (DWC2 201), wobei:
sich die erste Produktleitung zu dem Flash-Behälter (510) erstreckt, der mit einer fünften Leitung und einer sechsten Leitung verbunden ist, wobei sich die fünfte Leitung von der Oberseite des Flash-Behälters (510) zu einem Einlass der zweiten Trennwand-Destillationssäule (DWC2 201) erstreckt und sich die sechste Leitung von der Unterseite des Flash-Behälters (510) zu einem Einlass des Verdampfers (520) erstreckt.

15. Vorrichtung nach Anspruch 14, wobei der Verdampfer (520) mit einer siebten Leitung und einer dritten Rückführleitung verbunden ist, wobei sich die siebte Leitung von der Oberseite des Verdampfers (520) zu einem Einlass der zweiten Trennwand-Destillationssäule (DWC2 201) erstreckt und sich die dritte Rückführleitung von der Unterseite des Verdampfers (520) zu einem Einlass der ersten reaktiven Destillationssäule (210) erstreckt.

16. Vorrichtung nach einem der Ansprüche 13 bis 15, wobei das Dialkylcarbonat Dimethylcarbonat umfasst, das Diarylcarbonat Diphenylcarbonat umfasst, der aromatische Alkohol Phenol umfasst und der Alkylalkohol Methanol umfasst.

## Revendications

1. Procédé pour la production de carbonate de diaryle par la réaction d'un carbonate de dialkyle et d'un alcool aromatique en présence d'un catalyseur de transestérification, comprenant :
la production d'un premier flux supérieur (5) comprenant du carbonate de dialkyle et de l'alcool d'alkyle à partir d'une première colonne de distillation réactive (210) ;
l'introduction d'un flux de réactif (5') comprenant le premier flux supérieur (5) à une première colonne de distillation à paroi divisée (DWC1 200) et la séparation du mélange avec l'utilisation de la première colonne de distillation à paroi divisée (DWC1 200) ;
la récupération à partir de la première colonne de distillation réactive (210) d'un premier flux inférieur (6) comprenant du carbonate d'aryle d'alkyle ; et
l'introduction du premier flux inférieur (6) dans une deuxième colonne de distillation réactive (320) pour produire un deuxième flux inférieur (15) comprenant du carbonate de diaryle ;
la récupération à partir de la deuxième colonne de distillation réactive (320) du deuxième flux inférieur (15) comprenant du carbonate de diaryle et du carbonate d'aryle d'alkyle, et un deuxième flux supérieur (16) comprenant de l'alcool aromatique et du carbonate de dialkyle, et le recyclage du deuxième flux supérieur (16) à la première colonne de distillation réactive (210) ;
la récupération à partir de la première colonne de distillation réactive (210) d'un premier flux latéral (13) comprenant du carbonate de dialkyle, et l'introduction du premier flux latéral (13) à une première colonne de rectification (810) ;
la récupération à partir de la première colonne de rectification (810) d'un troisième flux inférieur (12) comprenant de l'éther d'aryle d'alkyle et un troisième flux supérieur (14) comprenant du carbonate de dialkyle et de l'alcool d'alkyle, et le recyclage du troisième flux supérieur (14) jusqu'à la première colonne de distillation réactive (210) ;
la récupération à partir de la première colonne de distillation à paroi divisée (DWC1 200) d'un quatrième flux supérieur azéotrope (431) et le recyclage du quatrième flux supérieur azéotrope (431) à la colonne de séparation de produit brut (160) ; et
la récupération d'un flux de recyclage de carbonate de dialkyle/quatrième flux inférieur (426) comprenant un carbonate de dialkyle purifié à partir de la première colonne de distillation à paroi divisée (DWC1 200) et le recyclage du flux recyclé de carbonate de dialkyle (426) à la première colonne de distillation réactive (210) ;
la récupération d'un deuxième flux (416) comprenant du carbonate de dialkyle, de l'eau et de l'alcanol à partir d'un côté de la colonne de distillation à paroi divisée (DWC1 200) et l'introduction du deuxième flux (416) dans un décanteur (610) ; et
la récupération à partir du décanteur (610) d'un troisième flux (418) comprenant du carbonate de dialkyle et le recyclage du troisième flux (418) à la première colonne de distillation à paroi divisée (DWC1 200).

2. Procédé selon la revendication 1, comprenant en outre
l'introduction du deuxième flux inférieur (15) à un ballon de flashing (510) ;
la récupération à partir du ballon de flashing (510) d'un cinquième flux inférieur (10) comprenant un catalyseur et du carbonate de diaryle, et un cinquième flux supérieur (9) comprenant du carbonate de diaryle, et du carbonate d'aryle d'alkyle et de l'alcool aromatique ;
l'introduction du cinquième flux inférieur (10) à un évaporateur (520) ; et
la récupération à partir de l'évaporateur (520) d'un sixième flux supérieur (11) comprenant du carbonate de diaryle et un sixième flux inférieur (20) comprenant le catalyseur et du carbonate de diaryle, et le recyclage du catalyseur à une première colonne de distillation réactive (210).

3. Procédé selon la revendication 2, comprenant en outre :
l'introduction du sixième flux supérieur (11) à une deuxième colonne de distillation à paroi divisée (DWC2 201) ;
la récupération à partir de la deuxième colonne de distillation à paroi divisée (DWC2 201) d'un septième flux supérieur (17) comprenant du carbonate d'alkyle d'aryle non réagi et de l'alcool aromatique, et le recyclage du septième flux supérieur (17) à la deuxième colonne de distillation réactive (320) ; et
la récupération à partir de la deuxième colonne de distillation à paroi divisée (DWC2 201) d'un flux de produit (24) comprenant du carbonate de diaryle.

4. Procédé selon la revendication 1, comprenant :
la récupération à partir du décanteur (610) d'un quatrième flux (421) comprenant de l'eau, de l'alcanol et du carbonate de dialkyle ;
l'introduction du quatrième flux (421) dans une colonne de distillation de récupération d'eau (630) ;
la récupération à partir de la colonne de distillation de récupération d'eau (630) d'un cinquième flux (424) comprenant de l'eau, de l'alcanol et du carbonate de dialkyle, et un sixième flux (422) comprenant de l'eau ; et le recyclage du cinquième flux (424) au décanteur (610).

5. Procédé selon l'une quelconque des revendications 1-4, dans lequel le carbonate de dialkyle comprend du carbonate de diméthyle, l'alcool d'alkyle comprend du méthanol, le flux de recyclage de carbonate de dialkyle (426) comprend du carbonate de diméthyle purifié avec des impuretés en très petites quantités, l'alcool aromatique comprend du phénol et le carbonate d'aryle d'alkyle comprend du carbonate de phényle de méthyle.

6. Procédé selon l'une quelconque des revendications 1-5, dans lequel la première colonne de distillation réactive (210) est maintenue à une température de 120 °C à 250 °C, et une pression sur une partie supérieure de la première colonne de distillation réactive (210) de 4 à 6 bars (400 à 600 kPa).

7. Procédé selon l'une quelconque des revendications 1-6, dans lequel la deuxième colonne de distillation réactive (320) est maintenue à une température de 140 °C à 240 °C, et une pression sur une partie supérieure de la deuxième colonne de distillation réactive (320) de 0,2 à 0,9 bars (20 à 90 kPa).

8. Procédé selon l'une quelconque des revendications 1-7, dans lequel la première colonne de rectification (810) est maintenue à une température de 100 °C à 190 °C, et une pression sur une partie supérieure de la colonne (810) de 1 à 3 bars (100 à 300 kPa).

9. Procédé selon l'une quelconque des revendications 1-8, dans lequel la première colonne de distillation à paroi divisée (DWC1 200) est maintenue à une température de 60°C à 140°C, et une pression sur une partie supérieure de la première colonne de distillation à paroi divisée (DWC1 200) de 1,2 à 3 bars (120 à 300 kPa).

10. Procédé selon la revendication 3, dans lequel la deuxième colonne de distillation à paroi divisée (DWC2 201) est maintenue à une température de 70 °C à 210 °C, et une pression sur une partie supérieure de la deuxième colonne de distillation à paroi divisée (DWC2 201) de 10 à 50 mbars (1 à 5 kPa).

11. Procédé selon l'une quelconque des revendications 1-10, dans lequel le flux de réactif (5') comprend en outre un flux de réactif effluent (412) orienté depuis une section de réaction (420) d'une installation de production de carbonate de dialkyle, qui sort d'une colonne de séparation de produit brut (160) dans une unité de production de carbonate de dialkyle et qui comprend du carbonate de dialkyle, de l'alcool d'alkyle et de l'eau.

12. Procédé selon l'une quelconque des revendications 1-11, comprenant en outre l'introduction du deuxième flux inférieur (15) à une section de purification (704) sans passer par une autre colonne de distillation réactive.

13. Appareil pour la production continue de carbonate de diaryle par la réaction d'un carbonate de dialkyle et d'un alcool aromatique en présence d'un catalyseur de transestérification, l'appareil comprenant : une première colonne de distillation réactive (210), une deuxième colonne de distillation réactive (320), une première colonne de rectification (810), une première colonne de distillation à paroi divisée (DWC1 200) et une pluralité de lignes pour transporter un réactif et des flux de produit ;
dans lequel la première colonne de distillation réactive (210) est reliée à des lignes d'entrée pour l'introduction de réactifs, et à une première ligne de transfert, une deuxième ligne de transfert et une troisième ligne de transfert, la première ligne de transfert s'étend de la partie supérieure de la première colonne de distillation réactive (210) à une entrée de la première colonne de distillation à paroi divisée (DWC1 200), la deuxième ligne de transfert s'étend de la partie inférieure de la première colonne de distillation réactive (210) à une entrée de la deuxième colonne de distillation réactive (320) sans passer par une autre colonne de distillation réactive, et la troisième ligne de transfert s'étend du côté de la première colonne de distillation réactive (210) à une entrée de la première colonne de rectification (810) ;
dans lequel la deuxième colonne de distillation réactive (320) est reliée à une première ligne de recyclage s'étend de la partie supérieure de la deuxième colonne de distillation réactive (320) à une entrée de la première colonne de distillation réactive (210), et une première ligne de produit s'étend de la partie inférieure de la deuxième colonne de distillation réactive (320) pour fournir du carbonate de diaryle ;
dans lequel la première colonne de rectification (810) est reliée à une quatrième ligne de transfert, la quatrième ligne de transfert s'étend de la partie supérieure de la première colonne de rectification (810) à une entrée de la première colonne de distillation réactive (210) ; et
dans lequel la première colonne de distillation à paroi divisée (DWC1 200) est reliée à une deuxième ligne de produit pour fournir du carbonate de dialkyle/azéotrope d'alcool d'alkyle s'étend de la partie supérieure de la première colonne de distillation à paroi divisée (DWC1 200) par le biais d'une colonne de séparation de produit brut (160) à une entrée de la première colonne de distillation réactive (210), une ligne tracée latérale de hauteur moyenne s'étend d'un milieu de la première colonne de distillation à paroi divisée (DWC1 200) à un décanteur (610), une troisième ligne qui s'étend du décanteur (610) à la première colonne de distillation à paroi divisée (DWC1 200), et une deuxième ligne de recyclage s'étend de la partie inférieure de la première colonne de distillation à paroi divisée (DWC1 200) à une entrée de la première colonne de distillation réactive (210).

14. Appareil selon la revendication 13, comprenant en outre un ballon de flashing (510), un évaporateur (520), et une deuxième colonne de distillation à paroi divisée (DWC2 201), dans lequel :
la première ligne de produit s'étend au ballon de flashing (510), qui est relié à une cinquième ligne et à une sixième ligne, la cinquième ligne s'étend de la partie supérieure du ballon de flashing (510) à une entrée de la deuxième colonne de distillation à paroi divisée (DWC2 201), et la sixième ligne s'étend de la partie inférieure du ballon de flashing (510) à une entrée de l'évaporateur (520).

15. Appareil selon la revendication 14, dans lequel l'évaporateur (520) est relié à une septième ligne et à une troisième ligne de recyclage, la septième ligne s'étend de la partie supérieure de l'évaporateur (520) à une entrée de la deuxième colonne de distillation à paroi divisée (DWC2 201) et la troisième ligne de recyclage s'étend de la partie inférieure de l'évaporateur (520) à une entrée de la première colonne de distillation réactive (210).

16. Appareil selon l'une quelconque des revendications 13-15, dans lequel le carbonate de dialkyle comprend du carbonate de diméthyle, le carbonate de diaryle comprend du carbonate de diphényle, l'alcool aromatique comprend du phénol, et l'alcool d'alkyle comprend du méthanol.
